# EUROPEAN PATENT SPECIFICATION

(11) **EP 3 947 408 B1**
(45) Date of publication and mention of the grant of the patent: **07.06.2023**
(21) Application number: 20718737.8
(22) Date of filing: 01.04.2020
(51) Int. Cl.: C07H 19/10, C07H 19/20, A61K 31/7068, A61K 31/7076, A61P 35/00, A61P 35/02

(54) **SALT OF TRIPHOSPHATE PHOSPHORAMIDATES OF NUCLEOTIDES AS ANTICANCER COMPOUNDS**
SALZE VON NUKLEOTID-PHOSPHORAMIDAT-TRIPHOSPHATE ALS ANTIKREBS-VERBINDUNGEN
SELS DE PHOSPHORAMIDATE TRIPHOSPHATES DE NUCLÉOTIDES COMME AGENTS CONTRE LE CANCER

(30) Priority: 01.04.2019 GB 201904544
(43) Date of publication of application: 09.02.2022
(73) Proprietor: Nucana PLC, Edinburgh EH12 9DT (GB)
(72) Inventor: GRIFFITH, Hugh, Edinburgh Scotland EH12 9DT (GB); SERPI, Michaela, Cardiff University, Redwood Building, King Edward VII Avenue, Cardiff South Glamorgan CF10 3NB (GB); PERTUSATI, Fabrizio, Cardiff University, Redwood Building, King edward VII Avenue, Cardiff South Glamorgan CF10 3NB (GB); SLUSARCZYK, Magdalena, Cardiff University, Redwood Building, King Edward VII Avenue, Cardiff South Glamorgan CF10 3NB (GB); DI CIANO, Samuele, Cardiff University, Redwood Building, King Edward VII Avenue, Cardiff South Glamorgan CF10 3NB (GB)
(74) Representative: HGF
(86) International application number: PCT/GB2020/050871
(87) International publication number: WO 2020/201751

(56) References cited:
- WO-A2-2016/099982
- UGO PRADERE ET AL: "Synthesis of Nucleoside Phosphate and Phosphonate Prodrugs", CHEMICAL REVIEWS, vol. 114, no. 18, 24 September 2014 (2014-09-24), pages 9154-9218, XP055203528, ISSN: 0009-2665, DOI: 10.1021/cr5002035
- JADD SHELTON ET AL: "Metabolism, Biochemical Actions, and Chemical Synthesis of Anticancer Nucleosides, Nucleotides, and Base Analogs", CHEMICAL REVIEWS, vol. 116, no. 23, 23 November 2016 (2016-11-23), pages 14379-14455, XP055568917, US ISSN: 0009-2665, DOI: 10.1021/acs.chemrev.6b00209
- GRACHEV M A ET AL: "A new system for ATP-metric immunoanalysis", FEBS LETTERS, ELSEVIER, AMSTERDAM, NL, vol. 162, no. 2, 17 October 1983 (1983-10-17), pages 266-269, XP025607414, ISSN: 0014-5793, DOI: 10.1016/0014-5793(83)80769-8 [retrieved on 1983-10-17]

## Description

The present invention provides compounds useful in the treatment of cancer, e.g. leukaemia. The compounds comprise a salt of a triphosphate phosphoramidate. The present invention also provides formulations of said compounds and uses of said compounds.

### BACKGROUND TO THE INVENTION

Nucleoside based drugs have become a powerful tool in the treatment of human disease. In the treatment of cancer, in particular, the use of nucleoside drugs such as gemcitabine, clofarabine, cytarabine, cladribine and fludarabine is widespread.

The effectiveness of all nucleoside drugs however can be limited by both inherent and acquired resistance mechanisms.

One way in which the efficacy of nucleoside drugs can be improved is by administration as a monophosphate phosphoramidate. Monophosphate phosphoramidates are prodrugs of monophosphorylated nucleotides and have been shown to be particularly potent therapeutic agents in the fields of both antivirals and oncology. These compounds appear to avoid many of the resistance mechanisms which limit the utility of the parent nucleosides (see, for example, 'Application of ProTide Technology to Gemcitabine: A Successful Approach to Overcome the Key Cancer Resistance Mechanisms Leads to a New Agent (NUC-1031) in Clinical Development'; Slusarczyk et al; J. Med. Chem.; 2014, 57, 1531-1542; McGuigan et al.; Phosphoramidate ProTides of the anticancer agent FUDR successfully deliver the preformed bioactive monophosphate in cells and confer advantage over the parent nucleoside; J. Med. Chem.; 2011, 54, 7247-7258; and Vande Voorde et al.; The cytostatic activity of NUC-3073, a phosphoramidate prodrug of 5-fluoro-2'-deoxyuridine, is independent of activation by thymidine kinase and insensitive to degradation by phosphorolytic enzymes; Biochem. Pharmacol.; 2011, 82, 441-452; WO2005/012327; WO2006/100439; WO2012/117246 and WO2016/083830). An exemplary monophosphate phosphoramidate is NUC-1031, a monophosphate phosphoramidate of gemcitabine:

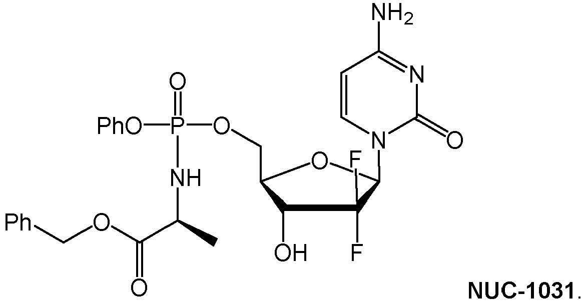

Whilst the monophosphate phosphoramidate strategy has proved to be very effective for certain nucleosides, it is not as effective with other nucleoside drugs. There is therefore a need to find further methods of potentiating nucleoside drug molecules.

Monophosphate phosphoramidates can also be poorly soluble in aqueous solvents and this can make administration challenging.

Pradere et al, Chem. Rev. 2014, 114, 18, 9154-9218, describes the synthesis of nucleoside phosphate and phosphonate prodrugs, including prodrugs of nucleoside mono-, di-, and tri-phosphates.

Shelton et al, Chem. Rev. 2016, 116, 23, 14379-14455, describes the chemical synthesis and biology of anticancer nucleoside, nucleotide, and base analogs that are FDA-approved and in clinical development since 2000. The cellular biology and clinical biology of analogs, drug resistance mechanisms, and compound specificity towards different cancer types are discussed. Analog syntheses, as well as improved and scale-up syntheses, are discussed.

WO 2016/099982 describes compounds for the treatment of infectious diseases and methods of treating such diseases. The compounds are derivatives of clevudine.

Grachev et al, FEBS Lett, 1983, 162 (2), 266-269, describes the treatment of amino-group-containing antigens with adenosine-5'-trimetaphosphate, which results in their chemical modification by -pppA residues. The reaction of ATP with adenosine-5'-trimetaphosphate to give an ATP γ-amidate is described.

It is an aim of certain embodiments of the present invention to provide a therapeutic agent that, in use, has therapeutic efficacy in the prophylaxis or treatment of cancer.

It is an aim of certain embodiments of the present invention to provide a therapeutic agent that, in use, has a greater therapeutic efficacy in the prophylaxis or treatment of cancer than the parent nucleoside or the corresponding monophosphate phosphoramidate.

It is an aim of certain embodiments of the present invention to provide a therapeutic agent that is more conveniently administered than the corresponding monophosphate phosphoramidate.

Certain embodiments of the present invention solve some or all of the above stated objects.

### STATEMENT OF THE INVENTION

In a first aspect of the invention there is provided a compound of formula (I), or a pharmaceutically acceptable salt thereof: 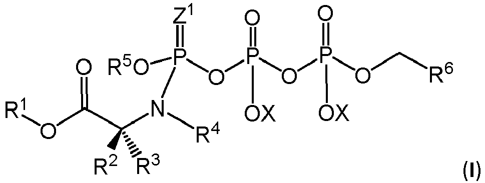 wherein
R¹ is independently at each occurrence selected from: C₁-C₂₄-alkyl, C₃-C₂₄-alkenyl, C₃-C₂₄-alkynyl, C₀-C₄-alkylene-C₃-C₈-cycloalkyl and C₀-C₄-alkylene-aryl;
R² and R³ are each independently at each occurrence selected from H, C₁-C₆-alkyl and C₁-C₃-alkylene-R⁷; or R² and R³ together with the atom to which they are attached form a 3- to 6-membered cycloalkyl or heterocycloalkyl group;
R⁴ is independently at each occurrence H or C₁-C₄-alkyl;
or R⁴, a group selected from R² and R³ and the atoms to which they are attached may form a 3- to 6-membered heterocycloalkyl group;
R⁵ is independently at each occurrence selected from aryl, 5-, 6-, 9- or 10-membered heteroaryl, C₃-C₈-cycloalkyl, 3- to 7-membered heterocycloalkyl, C₁-C₃-alkylene-R^{5a} and C₁-C₈-alkyl; said aryl being optionally fused to C₆-C₈-cycloalkyl;
R^{5a} is independently at each occurrence selected from aryl, 5-, 6-, 9- or 10-membered heteroaryl, C₃-C₈-cycloalkyl, 3- to 7-membered heterocycloalkyl, said aryl being optionally fused to C₃-C₃-cycloalkyl;
R⁶ is independently selected from: 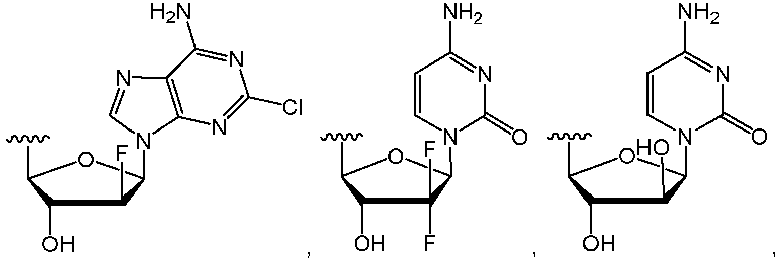 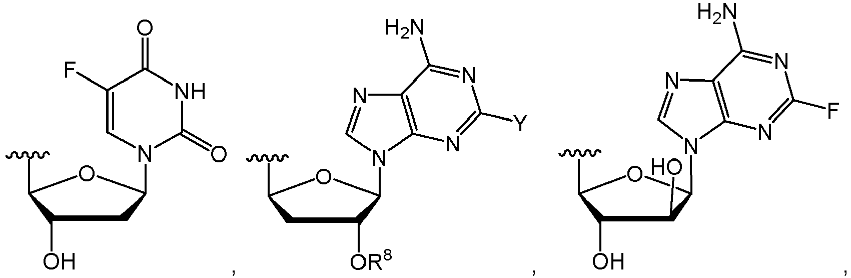 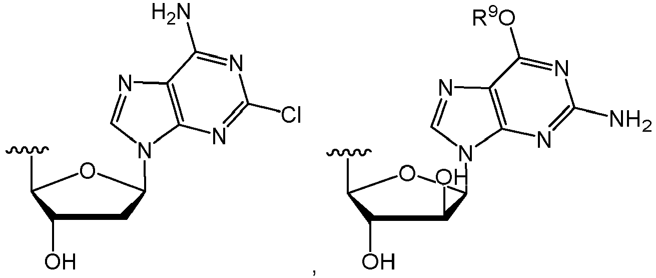 and 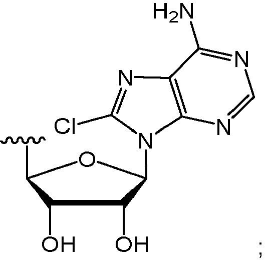
R⁷ is independently at each occurrence selected from aryl, imidazole, indole, SR^{a}, OR^{a}, CO₂R^{a}, CO₂NR^{a}R^{a}, NR^{a}R^{b} and NH(=NH)NH₂;
R⁸ is independently selected from H and 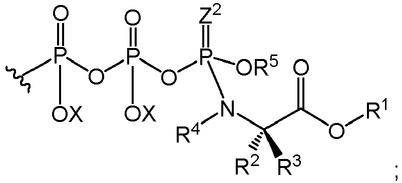
R⁹ is independently selected from H and Me;
Z¹ and Z² are each independently selected from O and S;
Y is independently selected from H, F, Cl and OMe;
X is at each occurrence selected from H and a pharmaceutically acceptable cation; provided that in at least one occurrence X is a pharmaceutically acceptable cation;
wherein any aryl group is either phenyl or naphthyl;
wherein where any of R¹, R², R³, R⁴, R⁵ or R⁷ is an alkyl, cycloalkyl, heterocycloalkyl, aryl or heteroaryl, that alkyl, cycloalkyl, heterocycloalkyl, aryl or heteroaryl group is optionally substituted with from 1 to 4 substituents selected from: halo, nitro, cyano, NR^{a}R^{a}, NR^{a}S(O)₂R^{a}, NR^{a}C(O)R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a}, C(O)R^{a}, CONR^{a}R^{a}, CR^{a}R^{a}NR^{a}R^{a}, C₁-C₄-alkyl, C₂-C₄-alkenyl, C₂-C₄-alkynyl and C₁-C₄-haloalkyl;
wherein R^{a} is independently at each occurrence selected from: H and C₁-C₄-alkyl; and R^{b} is independently at each occurrence selected from: H, C₁-C₄-alkyl and C(O)-C₁-C₄-alkyl.

The inventors have found that phosphoramidated triphosphates of formula (I) have good activities against a range of cancer cell lines, including solid tumour and haematological cancer cell lines. In certain cases the triphosphates of formula (I) are more active than the corresponding monophosphate phosphoramidate.

For the absence of doubt, where X is a pharmaceutically acceptable cation; the oxygen with which it is associated will be deprotonated, i.e. it will be an anion possessing a single negative charge.

The inventors have found that deprotonation of at least one phosphate group of triphosphate phosphoramidates provides high stability. The ionic nature of the compounds of the invention also means that they are more soluble in water than the corresponding monophosphate phosphoramidate.

A number of enzymatic processes are needed to convert a monophosphate phosphoramidate into the corresponding monophosphate nucleotide, including ester cleavage by carboxylesterases and cleavage of the resultant amino acid from the monophosphate nucleotide by phosphoramidase-type enzymes. The triphosphate phosphoramidates of the invention are larger molecules, having a different shape and they are ionic, casting doubt over whether they will interact with the relevant enzymes in the same way as monophosphate phosphoramidates for conversion to the triphosphate nucleotide. Nevertheless, the triphosphate phosphoramidates of formula (I) are still active in cells.

In embodiments, the compound of formula (I) is a compound of formula (II):

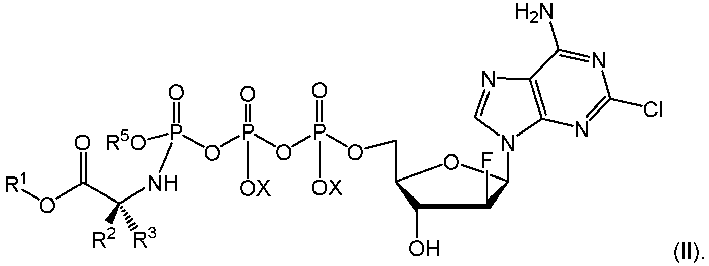

Triphosphate phosphoramidates of clofarabine such as those of formula (II) were more active than the corresponding monophosphate phosphoramidate against a number of cancer cell lines, including the haematological cell lines.

In embodiments, the compound of formula (I) is a compound of formula (III):

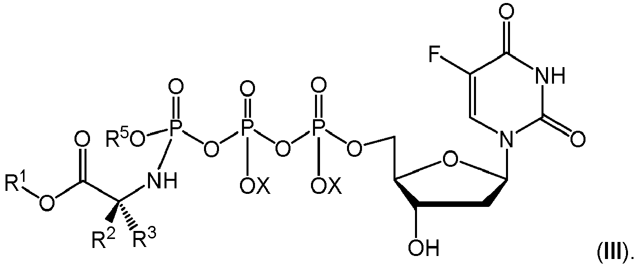

In embodiments, the compound of formula (I) is a compound of formula (IV):

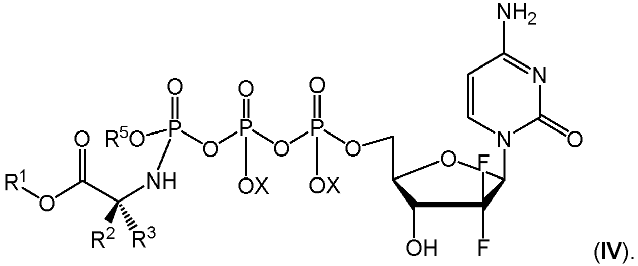

In embodiments, the compound of formula (I) is a compound of formula (V):

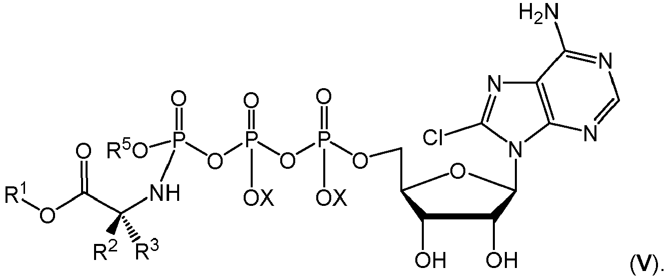

In embodiments, the compound of formula (I) is a compound of formula (VI):

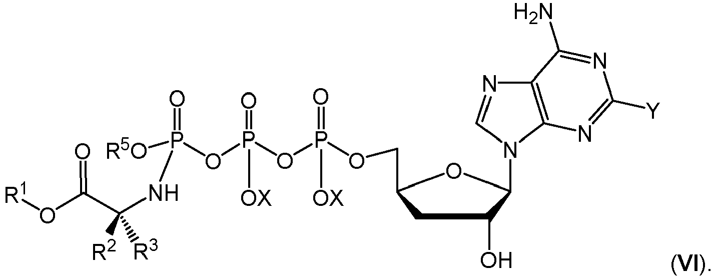

In embodiments, the compound of formula (I) is a compound of formula (VII): 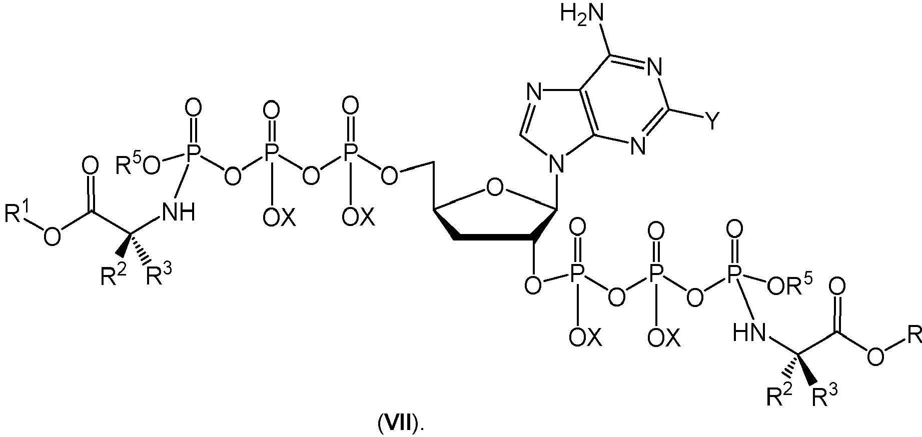 In these embodiments, it may be that R¹, R², R³, R⁵ and X are the same at both occurrences.

In embodiments, the compound of formula (I) is a compound of formula (**VIII**):

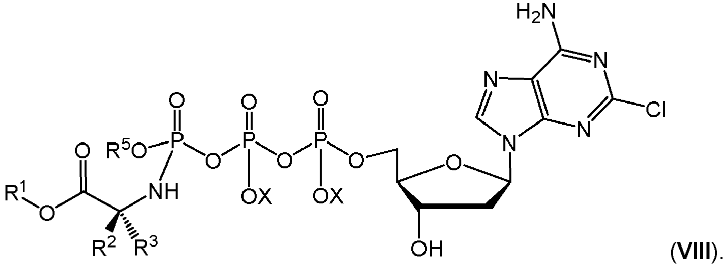

In embodiments, the compound of formula (**I**) is a compound of formula (IX):

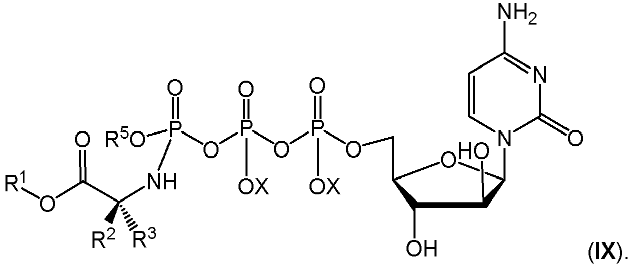

In embodiments, the compound of formula (**I**) is a compound of formula (X):

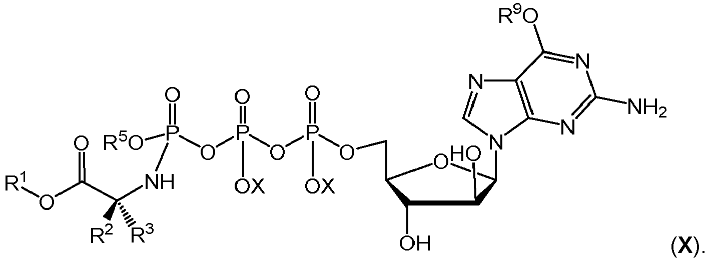

The following statements apply to compounds of any of formulae (**I**) to (X). These statements are independent and interchangeable. In other words, any of the features described in any one of the following statements may (where chemically allowable) be combined with the features described in one or more other statements below. In particular, where a compound is exemplified or illustrated in this specification, any two or more of the statements below which describe a feature of that compound, expressed at any level of generality, may be combined so as to represent subject matter which is contemplated as forming part of the disclosure of this invention in this specification.

R¹ may be independently at each occurrence selected from: C₁-C₂₄-alkyl, C₃-C₂₄-alkenyl, C₃-C₂₄-alkynyl, C₀-C₄-alkylene-C₃-C₆-cycloalkyl and C₀-C₄-alkylene-aryl.

R¹ may be independently at each occurrence selected from: C₁-C₂₄-alkyl, C₀-C₄-alkylene-Cs-Cs-cycloalkyl and CH₂-aryl. R¹ may be independently at each occurrence selected from: C₁-C₁₀-alkyl, C₄-C₆-cycloalkyl and benzyl. R¹ may be independently at each occurrence selected from: C₁-C₈-alkyl, C₆-cycloalkyl and benzyl.

R¹ may be C₁-C₈ alkyl.

R¹ may be selected such that it comprises three or more carbon atoms. R¹ may be selected such that it comprises five or more carbon atoms. R¹ may therefore be selected such that it includes six or more carbon atoms. R¹ is preferably selected such that it comprises only carbon and hydrogen atoms. R¹ may be selected from C₅-C₇-cycloalkyl, C₅-Cs-alkyl and benzyl, optionally wherein said groups are unsubstituted. R¹ may be unsubstituted benzyl. R¹ may be neopentyl. R¹ may be ethyl.

It may be that R³ is H. It may be that R³ is C₁-C₄-alkyl. It may be that R³ is methyl. It may be that R² is selected from C₁-C₆-alkyl and C₁-C₃-alkylene-R⁷. It may be that R² is C₁-C₃-alkylene-R⁷, R⁷ is SR^{a} and R^{a} is C₁-alkyl. It may be that R² is C₁-C₄-alkyl. It may be that R² is selected from methyl and isopropyl. R² may be methyl. R² may be H.

R⁴ is preferably H.

It may be that R⁴, a group selected from R² and R³, and the atoms to which they are attached form a 3- to 6-membered heterocycloalkyl group. It may be that R⁴, a group selected from R² and R³, and the atoms to which they are attached do not form a 3- to 6-membered heterocycloalkyl group. It may be that R² and R³ are each independently at each occurrence selected from H, C₁-C₆-alkyl and C₁-C₃-alkylene-R⁷; or R² and R³ together with the atom to which they are attached form a 3- to 6-membered heterocycloalkyl group; and R⁴ is independently at each occurrence H or C₁-C₄-alkyl.

It may be that R⁵ is substituted or unsubstituted phenyl, which may be optionally fused to a C₃-C₃-cycloalkyl ring, e.g. a cyclohexane ring. It may be that R⁵ is substituted or unsubstituted phenyl. It may be that R⁵ is substituted or unsubstituted naphthyl (e.g. 1-naphthyl). Preferably, R⁵ is selected from unsubstituted phenyl or unsubstituted naphthyl (e.g. 1-naphthyl). Thus, R⁵ may be unsubstituted phenyl. Alternatively, R⁵ may be unsubstituted naphthyl (e.g. 1-naphthyl). R⁵ may be C₁-C₆-alkyl, e.g. ethyl.

R⁶ may be independently selected from: 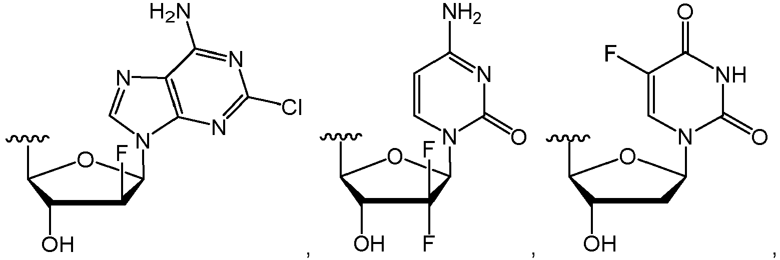 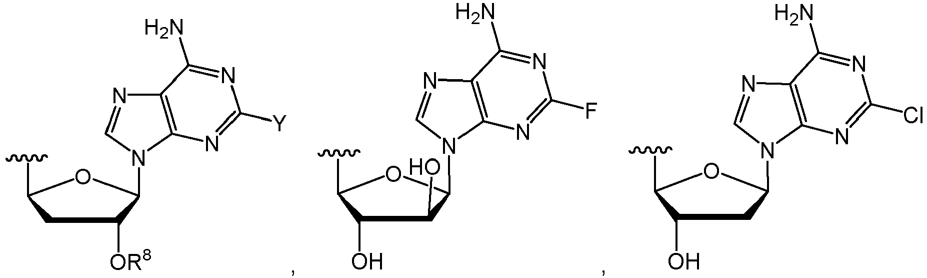 and

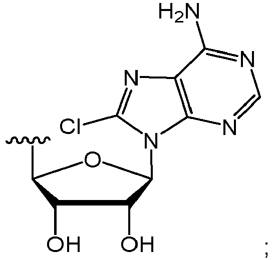

R⁶ may be

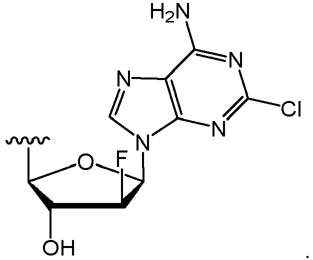

R⁶ may be

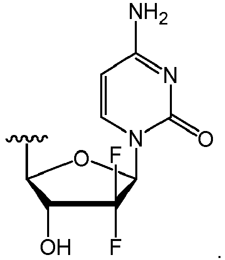

R⁶ may be

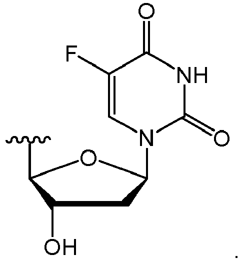

R⁶ may be

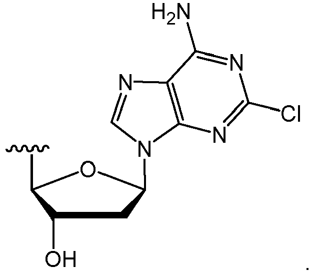

R⁶ may be 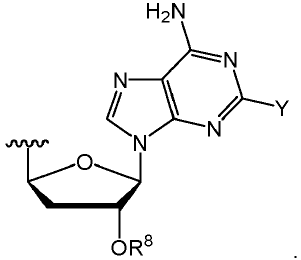 Y may be H. Y may be F. Y may be CI. Y may be OMe.

R⁸ may be H. R⁸ may be 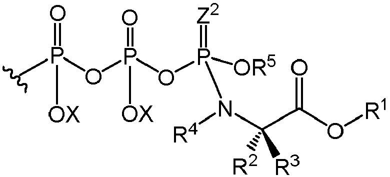 . Z² may be S. Z² may be O.

Where R⁸ is 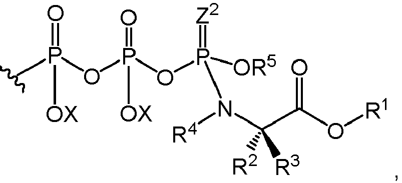 it may be that R¹, R², R³, R⁵, X and Z² are the same at both occurrences.

Where R⁸ is 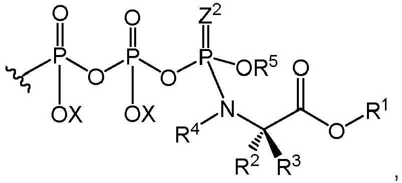 X will at two separate occurrences be a pharmaceutically acceptable cation. Thus, there is at least one deprotonated oxygen on each triphosphate group.

R⁶ may be

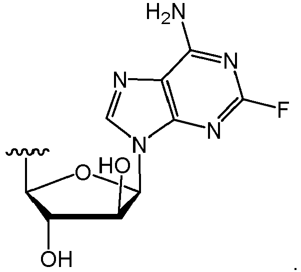

R⁶ may be

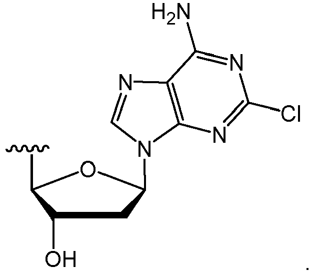

R⁶ may be

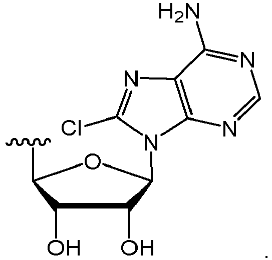

R⁶ may be

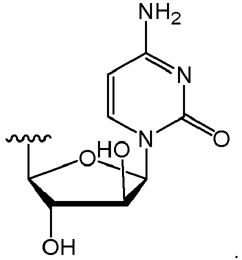

R⁶ may be 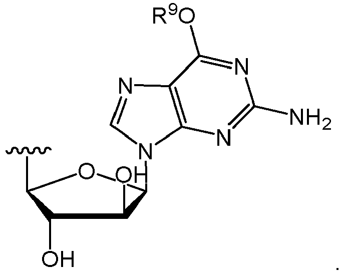 R⁹ may be H. R⁹ may be Me.

The compound of formula (I) may be selected from:

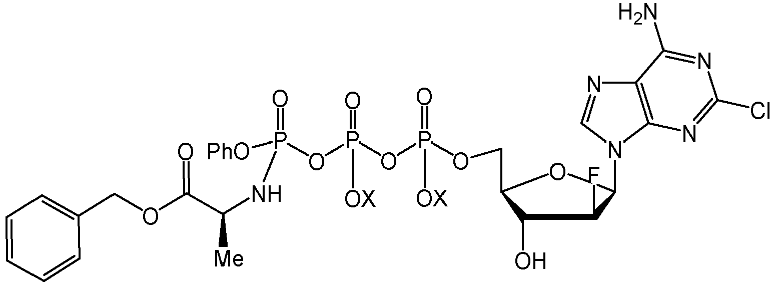

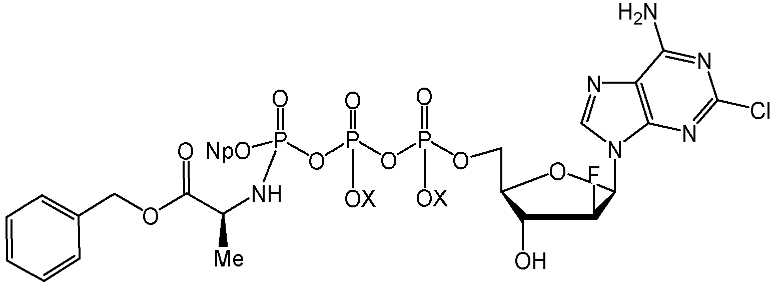

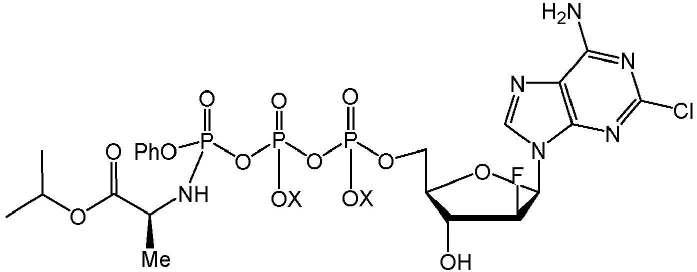

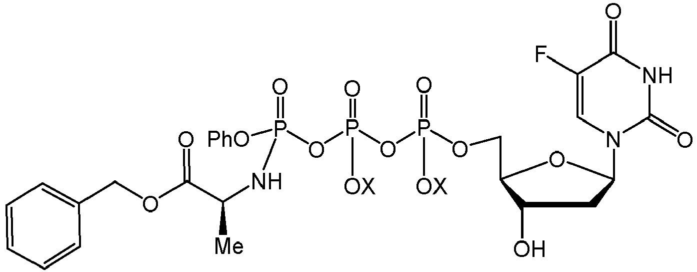

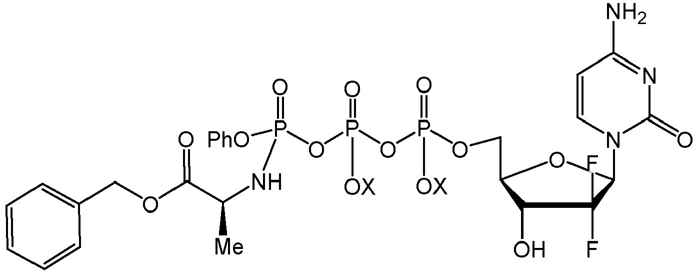

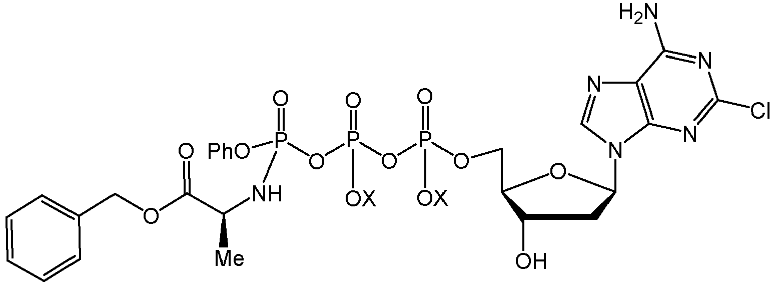

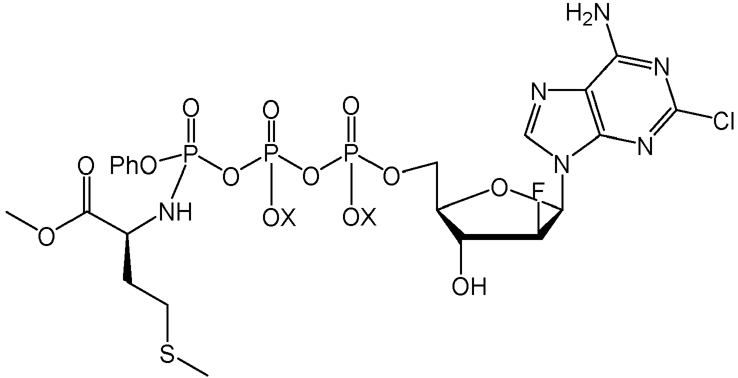

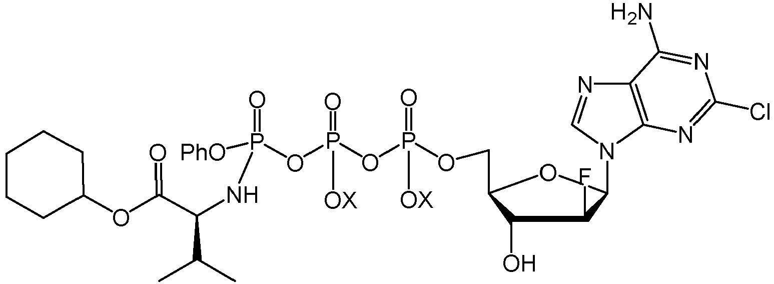

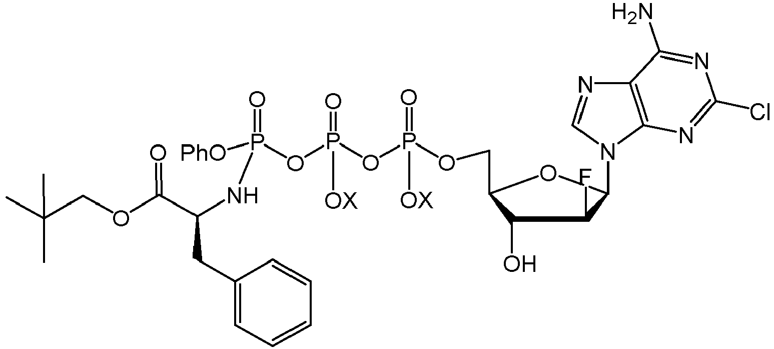

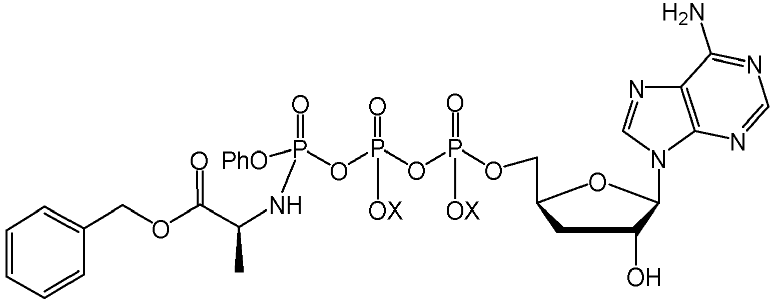

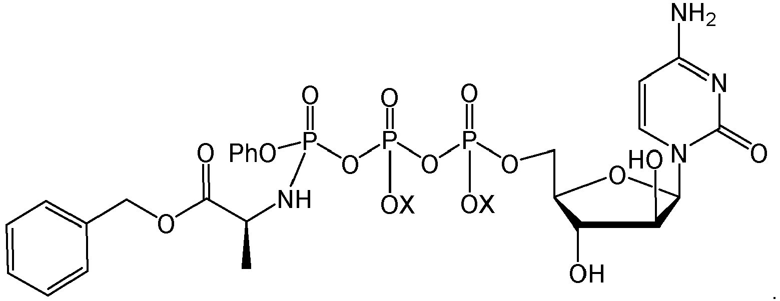

It may be that X is at each occurrence a pharmaceutically acceptable cation. It may be that X is at each occurrence the same pharmaceutically acceptable cation.

It may be that X is at one occurrence a H and at one occurrence a pharmaceutically acceptable cation. Where the compound comprises two triphosphate groups, it may be that each triphosphate group comprises one X that is H and one X that is a pharmaceutically acceptable cation.

X may be a metal cation or it may be an ammonium cation. X may be a metal cation, e.g. a cation of an alkali or alkali earth metal. X may be an ammonium cation, e.g. a trialkylammonium cation or an ammonium cation of a nitrogen heterocycle. Illustrative cations include those derived from aluminium, arginine, benzathine, calcium, choline, diethylamine, diolamine, glycine, lysine, magnesium, meglumine, olamine, potassium, sodium, tromethamine, triethylamine and zinc cations. X may be a triethylamine cation.

Compounds of the invention comprise a chiral centre at the phosphorus atom (γ)-phosphorus) that is bonded to OR⁵. The compound may be present as a mixture of phosphate diastereoisomers, as the (*S*)-epimer at the phosphorus atom in substantially diastereomerically pure form or as the (*R*)-epimer at the phosphorus atom in substantially diastereomerically pure form. 'Substantially diastereomerically pure' is defined for the purposes of this invention as a diastereomeric purity of greater than about 90%. If present as a substantially diastereoisomerically pure form, the compound may have a diastereoisomeric purity of greater than 95%, 98%, 99%, or even 99.5%. Alternatively, the compound may be present as a mixture of phosphate diastereoisomers.

The (*R*)- and/or (*S*)-epimers of the compound can be obtained in substantially diastereomerically pure form by chromatography, e.g. HPLC optionally using a chiral column. Alternatively, the (*R*)- and/or (*S*)-epimers of the compound can be obtained in substantially diastereomerically pure form by crystallisation from an appropriate solvent or solvent system.

According to a second aspect of the present invention, there is provided a compound of formula (I), or a pharmaceutically acceptable salt thereof, for use in a method of treatment.

According to a third aspect of the present invention, there is provided a compound of formula (I), or a pharmaceutically acceptable salt thereof, for use in the prophylaxis or treatment of cancer.

According to a fourth aspect of the present invention there is provided use of a compound of formula (I), or a pharmaceutically acceptable salt thereof, in the manufacture of a medicament for the prophylaxis or treatment of cancer.

With respect to each of the third and fourth aspects of the present invention, embodiments of the invention comprise a cancer selected from but not restricted to the group consisting of: pancreatic cancer, bladder cancer, other urothelial cancers (e.g. cancers of ureter and renal pelvis), gastrointestinal cancers (also known as cancer of the digestive tract, including: oesophageal cancer, gastric cancer, stomach cancer, bowel cancer, small intestine cancer, colon cancer, colorectal cancer, appendix mucinous, goblet cell carcinoid, liver cancer, biliary cancer, gallbladder cancer, anal cancer and rectal cancer), lung cancer, renal (or kidney) cancer, biliary cancer, prostate cancer, cholangiocarcinoma, neuroendocrine cancer, sarcoma, lymphoma, thymic cancer, glioblastoma multiforme, a cancer of an unknown primary origin, mesothelioma, adrenal cancer, testicular cancer, cancer of the central nervous system, basal cell carcinoma, Bowens disease, other skin cancers (such as malignant melanoma, merckel cell tumour and rare appendage tumours), ocular surface squamous neoplasia, germ cell tumours, leukaemia, multiple myeloma, lung cancer, liver cancer, breast cancer, head and neck cancer, neuroblastoma, thyroid carcinoma, oral squamous cell carcinoma, urinary bladder cancer, Leydig cell tumour, and gynaecological cancers (including ovarian cancer, cancer of the fallopian tube, peritoneal cancer, endometrial cancer, uterine cancer and cervical cancer, including epithelia cervix carcinoma).

In certain embodiments, the cancer is a leukaemia or a lymphoma.

The cancer may be a leukaemia.

There are four main types of leukaemia depending on whether they are chronic or acute, and myeloid or lymphoid in origin. These are: acute myeloblastic leukaemia (AML), acute lymphoblastic leukaemia (ALL), chronic myelogenous leukaemia (CML) and chronic lymphocytic leukaemia (CLL).

Mixtures of these are also known; e.g. biphenotypic acute leukaemia (BAL; which is a mix of AML and ALL).

In particular embodiments, the leukaemia is selected from the group consisting of: acute myeloblastic leukaemia (AML), acute lymphoblastic leukaemia (ALL), chronic myelogenous leukaemia (CML), chronic lymphocytic leukaemia (CLL) and biphenotypic acute leukaemia (BAL; which is a mix of AML and ALL).

Chronic lymphocytic leukaemia (CLL) includes the subtypes: B-cell CLL (B-CLL), B-cell prolymphocytic leukaemia (PLL) and T-cell chronic prolymphocytic leukaemia (T-PLL) and several subtypes that differ at the genetic level.

Acute lymphoblastic leukaemia (ALL) includes the subtypes: precursor B-cell ALL (B-ALL), precursor T-cell ALL (T-ALL), Burkitt-type ALL and Philadelphia chromosome positive (BCR-ABL fusion) ALL.

Acute myeloblastic leukaemia (AML) includes the subtypes: myeloid leukaemia, monocytic leukaemia and acute promyelocytic leukaemia (APL).

Chronic myelogenous leukaemia (CML) includes the subtypes: chronic granulocytic leukaemia (CGL) (CGL), Juvenile CML (JCML), chronic neutrophilic leukaemia (CNL), chronic myelomonocytic leukaemia (CMML) and atypical CML (aCML).

In particular embodiments, any of the above sub-types of CLL, ALL, AML and CML are suitable for treatment with the compounds of the invention or pharmaceutical compositions containing them

Other forms of leukemia that can be considered to sit outside of these four main groups include: erythroleukemia, arising from red blood cell precursors; and a lymphoma that has gone into the blood.

The cancer may be a lymphoma, e.g. a solid lymphoma.

There are two main types of lymphoma: Hodgkin's lymphoma and non-Hodgkin's lymphoma. Within each of these there are various subtypes.

In particular embodiments, the lymphoma is selected from the group consisting of: Hodgkin's lymphoma and non-Hodgkin's lymphoma.

In particular embodiments, the lymphoma is selected from the group consisting of: Burkitt's lymphoma (BL), mantle cell lymphoma (MCL), follicular lymphoma (FL), small lymphocytic lymphoma (SLL) indolent B-cell non-Hodgkin's lymphoma, histiocytic lymphoma (aka immunoblastic lymphoma; IBL) and diffuse large B-cell lymphoma (DLBCL), including activated-cell like diffuse large B-cell lymphoma (DLBCL-ABC) and germinal center B-cell like diffuse large B-cell lymphoma (DLBCL-GCB).

In certain embodiments, the cancer is a gastrointestinal cancer and can be selected from the group consisting of: oesophageal cancer, gastric cancer, stomach cancer, bowel cancer, small intestine cancer, colon cancer, colorectal cancer, appendix mucinous, goblet cell carcinoid, liver cancer, biliary cancer, gallbladder cancer, anal cancer and rectal cancer.

In certain embodiments, the cancer is of gynaecological origin and can be selected from the group consisting of: a cancer of the uterus, cancer of the fallopian tube, cancer of the endometrium, cancer of the ovary, cancer of the peritoneum and cancer of the cervix). Suitably the ovarian cancer may be epithelial ovarian cancer. Suitably the peritoneal cancer may be primary peritoneal cancer.

In particular, the cancer may be selected from, but not restricted to pancreatic cancer, lung cancer, bladder cancer, breast cancer, biliary cancer, lymphoma, a leukaemia, a gastrointestinal cancer and a gynaecological cancer.

The compounds of the invention have been found to retain activity even under hypoxic conditions. Cancers particularly associated with hypoxia are pancreatic and renal (or kidney) cancers.

The cancer may be relapsed. The cancer may be metastatic. The cancer may be previously untreated.

The cancer may be refractory cancer that has previously been treated but has proven unresponsive to prior treatment. Alternatively, the cancer patient may be intolerant of a previous therapy, for example, may develop side effects that make the patient intolerant to further treatment with the agent being administered.

According to a fifth aspect of the present invention, there is provided a pharmaceutical composition comprising a compound of formula (I), or a pharmaceutically acceptable salt thereof, and at least one pharmaceutically acceptable excipient. The pharmaceutical composition may be for use in the prophylaxis or treatment of cancer, e.g. a cancer or group of cancers mentioned above.

### DETAILED DESCRIPTION

The term 'saline' is intended to refer to an aqueous solution of sodium chloride. Saline solutions of the present invention will typically be sterile and will typically be at a concentration suitable for use in parenteral administration. Suitable concentrations are up to 2 w/v% or up to 1 w/v%. To optimise osmolarity different concentrations of saline can be used in the formulations of the invention, e.g. 0.9% or 0.45%.

The formulations of the present invention can be used in the treatment of the human body. They may be used in the treatment of the animal body.

The compounds in the formulations of the invention may be obtained, stored and/or administered in the form of a pharmaceutically acceptable salt. Suitable pharmaceutically acceptable salts include, but are not limited to, salts of pharmaceutically acceptable inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumaric, malic, citric, lactic, mucic, gluconic, benzoic, succinic, oxalic, phenylacetic, methanesulphonic, toluenesulphonic, benzenesulphonic, salicylic, sulphanilic, aspartic, glutamic, edetic, stearic, palmitic, oleic, lauric, pantothenic, tannic, ascorbic and valeric acids. Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulfate, hemioxalate and hemicalcium salts.

For the above-mentioned formulations of the invention the dosage administered will, of course, vary with the compound employed, the precise mode of administration, the treatment desired and the disorder indicated. Dosage levels, dose frequency, and treatment durations of compounds of the invention are expected to differ depending on the formulation and clinical indication, age, and co-morbid medical conditions of the patient. The size of the dose for therapeutic purposes of compounds of the invention will naturally vary according to the nature and severity of the conditions, the age and sex of the animal or patient and the route of administration, according to well known principles of medicine.

A pharmaceutical formulation typically takes the form of a composition in which active compounds, or pharmaceutically acceptable salts thereof, are in association with a pharmaceutically acceptable adjuvant, diluent or carrier. One such pharmaceutically acceptable adjuvant, diluent or carrier in the formulations of the invention is the polar aprotic solvent. Conventional procedures for the selection and preparation of suitable pharmaceutical formulations are described in, for example, "Pharmaceuticals - The Science of Dosage Form Designs", M. E. Aulton, Churchill Livingstone, 1988.

The formulations may be suitable for topical application (e.g. to the skin or bladder), for oral administration or for parenteral (e.g. intravenous administration).

Any solvents used in pharmaceutical formulations of the invention should be pharmaceutical grade, by which it is meant that they have an impurity profile which renders them suitable for administration (e.g. intravenous administration) to humans.

For oral administration the formulations of the invention may comprise the active compound admixed with an adjuvant or a carrier, for example, lactose, saccharose, sorbitol, mannitol; a starch, for example, potato starch, corn starch or amylopectin; a cellulose derivative; a binder, for example, gelatine or polyvinylpyrrolidone; and/or a lubricant, for example, magnesium stearate, calcium stearate, polyethylene glycol, a wax, paraffin, and the like, and then compressed into tablets. If coated tablets are required, the cores, prepared as described above, may be coated with a concentrated sugar solution which may contain, for example, gum arabic, gelatine, talcum and titanium dioxide. Alternatively, the tablet may be coated with a suitable polymer dissolved in a readily volatile organic solvent.

For the preparation of soft gelatine capsules, the active compounds may be admixed with, for example, a vegetable oil or polyethylene glycol. Hard gelatine capsules may contain granules of the compound using either the above-mentioned excipients for tablets. Also liquid or semisolid formulations of the active compounds may be filled into hard gelatine capsules.

Liquid preparations for oral application may be in the form of syrups or suspensions, for example, solutions containing the compound of the invention, the balance being sugar and a mixture of ethanol, water, glycerol and propylene glycol. Optionally such liquid preparations may contain colouring agents, flavouring agents, sweetening agents (such as saccharine), preservative agents and/or carboxymethylcellulose as a thickening agent or other excipients known to those skilled in art.

The formulations may be for parenteral (e.g. intravenous) administration. For parenteral (e.g. intravenous) administration the active compounds may be administered as a sterile aqueous or oily solution. Preferably, the active compounds are administered as a sterile aqueous solution. The aqueous solution may further comprise at least one surfactant and/or organic solvent. Illustrative organic solvents include dimethylacetamide, ethanol, ethyleneglycol, *N*-methyl-pyrrolidinone, dimethylsulfoxide, dimethylformamide and isopropanol. Illustrative surfactants include polyethoxylated fatty acids and fatty acid esters and mixtures thereof. Suitable surfactants include polyethoxylated castor oil (e.g. that sold under the trade name Kolliphor^{®} ELP); or polyethoxylated stearic acid (e.g. that sold under the trade names Solutol^{®} or Kolliphor^{®} HS15); or polyethoxylated (e.g. polyoxyethylene (20)) sorbitan monooleate, (e.g. that are sold under the trade names Polysorbate 80 or Tween^{®} 80).

The pharmaceutical composition of the invention will preferably comprise from 0.05 to 99 %w (per cent by weight) compound of the invention, more preferably from 0.05 to 80 %w compound of the invention, still more preferably from 0.10 to 70 %w compound of the invention, and even more preferably from 0.10 to 50 %w compound of the invention, all percentages by weight being based on total composition.

Cyclodextrins have been shown to find wide application in drug delivery (Rasheed et al, Sci. Pharm., 2008, 76, 567-598). Cyclodextrins are a family of cyclic oligosaccharides. They act as a 'molecular cage' which encapsulates drug molecules and alters properties of those drug molecules such as solubility. Cyclodextrins comprise (α-1,4)-linked α-D-glucopyranose units. Cyclodextrins may contains 6, 7 or 8 glucopyranose units (designated α-, β- and γ-cyclodextrins respectively). Cyclodextrins used in pharmaceutical formulations are often β-cyclodextrins. The pendant hydroxyl groups can be alkylated with a C₁-C₆ substituted or unsubstituted alkyl group. Examples of cyclodextrins are α-cyclodextrin, β-cyclodextrin, γ-cyclodextrin, 2-hydroxypropyl-β-cyclodextrin (HP-β-CD), sulfobutylether β-cyclodextrin sodium salt, partially methylated β-cyclodextrin. The formulations of the invention may also comprise at least one cyclodextrin.

The term Cₘ-Cₙ refers to a group with m to n carbon atoms.

The term "alkyl" refers to a linear or branched hydrocarbon group. An alkyl group is monovalent. For example, C₁-C₆-alkyl may refer to methyl, ethyl, *n*-propyl, *iso*-propyl, *n-*butyl, *sec*-butyl, *tert*-butyl, *n*-pentyl and *n*-hexyl. The alkyl groups are preferably unsubstituted.

The term "alkylene" refers to a linear hydrocarbon chain. An alkylene group is divalent. For example, C₁₋alkylene may refer to a CH₂ group. C₂-alkylene may refer to - CH₂CH₂- group. The alkylene groups are preferably unsubstituted.

The term "haloalkyl" refers to a hydrocarbon chain substituted with at least one halogen atom independently chosen at each occurrence from: fluorine, chlorine, bromine and iodine. The halogen atom may be present at any position on the hydrocarbon chain. For example, C₁-C₄-haloalkyl may refer to chloromethyl, fluoromethyl, trifluoromethyl, chloroethyl e.g. 1-chloromethyl and 2-chloroethyl, trichloroethyl e.g. 1,2,2-trichloroethyl, 2,2,2-trichloroethyl, fluoroethyl e.g. 1-fluoromethyl and 2-fluoroethyl, trifluoroethyl e.g. 1,2,2-trifluoroethyl and 2,2,2-trifluoroethyl, chloropropyl, trichloropropyl, fluoropropyl, trifluoropropyl. A halo alkyl group may be a fluoroalkyl group, i.e. a hydrocarbon chain substituted with at least one fluorine atom.

The term "alkenyl" refers to a branched or linear hydrocarbon chain containing at least one carbon-carbon double bond. The double bond(s) may be present as the *E* or *Z* isomer. The double bond may be at any possible position of the hydrocarbon chain. For example, "C₂-C₄-alkenyl" may refer to ethenyl, allyl and butenyl. The alkenyl groups are preferably unsubstituted.

The term "alkynyl" refers to a branched or linear hydrocarbon chain containing at least one carbon-carbon triple bond. The triple bond may be at any possible position of the hydrocarbon chain. For example, "C₂-C₆-alkynyl" may refer to ethynyl, propynyl, butynyl. The alkynyl groups are preferably unsubstituted.

The term "cycloalkyl" refers to a saturated hydrocarbon ring system containing 3, 4, 5 or 6 carbon atoms. For example, "3- to 6- membered cycloalkyl" may refer to cyclopropyl, cyclobutyl, cyclopentyl, cyclohexyl. The cycloalkyl groups are preferably unsubstituted.

The term "heterocycloalkyl" may refer to a saturated or partially saturated monocyclic group comprising 1 or 2 heteroatoms independently selected from O, S and N in the ring system (in other words 1 or 2 of the atoms forming the ring system are selected from O, S and N). Examples of heterocycloalkyl groups include; piperidine, piperazine, morpholine, thiomorpholine, pyrrolidine, tetrahydrofuran, tetrahydrothiophene, tetrahydropyran, dihydropyran, dioxane, azepine. The heterocycloalkyl groups are preferably unsubstituted or substituted.

The present invention includes tautomers, where tautomerisation is possible, of the compounds of formulae (**I**)-(**X**).

The present invention also includes formulations of all pharmaceutically acceptable isotopically-labelled forms of compound wherein one or more atoms are replaced by atoms having the same atomic number, but an atomic mass or mass number different from the atomic mass or mass number of the predominant isotope usually found in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds, for example, those incorporating a radioactive isotope, are useful in drug and/or substrate tissue distribution studies. The radioactive isotopes tritium, i.e. ³H, and carbon-14, i.e. ¹⁴C, and ¹⁸F are particularly useful for this purpose in view of their ease of incorporation and ready means of detection.

Substitution with heavier isotopes such as deuterium, i.e. ²H, may afford certain therapeutic advantages resulting from greater metabolic stability, for example, increased *in vivo* half-life or reduced dosage requirements, and hence may be preferred in some circumstances.

Isotopically-labelled compounds can generally be prepared by conventional techniques known to those skilled in the art or by processes analogous to those described using an appropriate isotopically-labelled reagent in place of the non-labelled reagent previously employed.

The formulation for use in the treatment of cancer including lymphoma or leukaemia, may involve, in addition to the formulations of the invention, conventional surgery or radiotherapy or chemotherapy. Such chemotherapy may include the administration of one or more other active agents.

Where a further active agent is administered as part of a method of treatment, such combination treatment may be achieved by way of the simultaneous, sequential or separate dosing of the individual components of the treatment. Such combination products employ the compounds of this invention within a therapeutically effective dosage range described hereinbefore and the one or more other pharmaceutically-active agent(s) within its approved dosage range.

Thus, the pharmaceutical formulations of the invention may comprise another active agent.

The one or more other active agents may be one or more of the following categories of anti-tumour agents:
(i) antiproliferative/antineoplastic drugs and combinations thereof, such as alkylating agents (for example cyclophosphamide, nitrogen mustard, bendamustin, melphalan, chlorambucil, busulphan, temozolamide and nitrosoureas); antimetabolites (for example gemcitabine and antifolates such as fluoropyrimidines like 5-fluorouracil and tegafur, raltitrexed, methotrexate, pemetrexed, cytosine arabinoside, and hydroxyurea); antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere and polokinase inhibitors); proteasome inhibitors, for example carfilzomib and bortezomib; interferon therapy; and topoisomerase inhibitors (for example epipodophyllotoxins like etoposide and teniposide, amsacrine, topotecan, mitoxantrone and camptothecin);
(ii) cytostatic agents such as antiestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) anti-invasion agents, for example dasatinib and bosutinib (SKI-606), and metalloproteinase inhibitors, inhibitors of urokinase plasminogen activator receptor function or antibodies to Heparanase;
(iv) inhibitors of growth factor function: for example such inhibitors include growth factor antibodies and growth factor receptor antibodies, for example the anti-erbB2 antibody trastuzumab [Herceptin^{™}], the anti-EGFR antibody panitumumab, the anti-erbB1 antibody cetuximab, tyrosine kinase inhibitors, for example inhibitors of the epidermal growth factor family (for example EGFR family tyrosine kinase inhibitors such as gefitinib, erlotinib and 6-acrylamido-*N*-(3-chloro-4-fluorophenyl)-7-(3-morpholinopropoxy)-quinazolin-4-amine (Cl 1033), erbB2 tyrosine kinase inhibitors such as lapatinib); inhibitors of the hepatocyte growth factor family; inhibitors of the insulin growth factor family; modulators of protein regulators of cell apoptosis (for example Bcl-2 inhibitors); inhibitors of the platelet-derived growth factor family such as imatinib and/or nilotinib (AMN107); inhibitors of serine/threonine kinases (for example Ras/Raf signalling inhibitors such as farnesyl transferase inhibitors, for example sorafenib , tipifarnib and lonafarnib), inhibitors of cell signalling through MEK and/or AKT kinases, c-kit inhibitors, abl kinase inhibitors, PI3 kinase inhibitors, Plt3 kinase inhibitors, CSF-1R kinase inhibitors, IGF receptor, kinase inhibitors; aurora kinase inhibitors and cyclin dependent kinase inhibitors such as CDK2 and/or CDK4 inhibitors;
(v) antiangiogenic agents such as those which inhibit the effects of vascular endothelial growth factor, [for example the anti-vascular endothelial cell growth factor antibody bevacizumab (Avastin^{™}); thalidomide; lenalidomide; and for example, a VEGF receptor tyrosine kinase inhibitor such as vandetanib, vatalanib, sunitinib, axitinib and pazopanib;
(vi) gene therapy approaches, including for example approaches to replace aberrant genes such as aberrant p53 or aberrant BRCA1 or BRCA2;
(vii) immunotherapy approaches, including for example adoptive cell transfer, such as CAR T-cell therapy; antibody therapy such as alemtuzumab, rituximab, ibritumomab tiuxetan (Zevalin^{®}) and ofatumumab; interferons such as interferon α; interleukins such as IL-2 (aldesleukin); interleukin inhibitors for example IRAK4 inhibitors; cancer vaccines including prophylactic and treatment vaccines such as HPV vaccines, for example Gardasil, Cervarix, Oncophage and Sipuleucel-T (Provenge); and toll-like receptor modulators for example TLR-7 or TLR-9 agonists;
(viii) cytotoxic agents for example fludarabine (fludara), cladribine, pentostatin (Nipent^{™});
(ix) steroids such as corticosteroids, including glucocorticoids and mineralocorticoids, for example aclometasone, aclometasone dipropionate, aldosterone, amcinonide, beclomethasone, beclomethasone dipropionate, betamethasone, betamethasone dipropionate, betamethasone sodium phosphate, betamethasone valerate, budesonide, clobetasone, clobetasone butyrate, clobetasol propionate, cloprednol, cortisone, cortisone acetate, cortivazol, deoxycortone, desonide, desoximetasone, dexamethasone, dexamethasone sodium phosphate, dexamethasone isonicotinate, difluorocortolone, fluclorolone, flumethasone, flunisolide, fluocinolone, fluocinolone acetonide, fluocinonide, fluocortin butyl, fluorocortisone, fluorocortolone, fluocortolone caproate, fluocortolone pivalate, fluorometholone, fluprednidene, fluprednidene acetate, flurandrenolone, fluticasone, fluticasone propionate, halcinonide, hydrocortisone, hydrocortisone acetate, hydrocortisone butyrate, hydrocortisone aceponate, hydrocortisone buteprate, hydrocortisone valerate, icomethasone, icomethasone enbutate, meprednisone, methylprednisolone, mometasone paramethasone, mometasone furoate monohydrate, prednicarbate, prednisolone, prednisone, tixocortol, tixocortol pivalate, triamcinolone, triamcinolone acetonide, triamcinolone alcohol and their respective pharmaceutically acceptable derivatives. A combination of steroids may be used, for example a combination of two or more steroids mentioned in this paragraph;
(x) targeted therapies, for example PI3Kd inhibitors, for example idelalisib and perifosine; or checkpoint inhibitor compounds including anti-PD-1, anti- PD-L1 and anti-CTLA4 molecules, such as nivolumab, pembrolizumab, pidilizumab, atezolizumab, durvalumab and avelumab.

The one or more other active agents may also be antibiotic.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification.

### EXAMPLES

**Throughout the specification, these abbreviations have the following meanings.**

| | | | |
|---|---|---|---|
| aq | aqueous | MS | mass spectrometry |
| Bz | benzoyl | NMR | nuclear magnetic resonance |
| CDI | carbonyldiimidazole | Np | 1-naphthyl |
| DCM | dichloromethane | Py | pyridine |
| DMF | N,N-dimethyl formamide | rt | room temperature |
| DMSO | dimethylsulphoxide | TBAF | tetra-n-butylammonium fluoride |
| DMTr | 4,4-dimethoxytriphenylmethyl | TBDMSCI | tert-butyldimethylsilyl chloride |
| EC₅₀ | half maximal effective concentration | TEAB | triethylammonium hydrogen carbonate buffer |
| eq. | equivalents | TFA | Trifluoroacetic acid |
| HPLC | high pressure liquid chromatography | *t*_{R} | retention time |
| h | hours | Ts | p-toluenesulphonyl |
| IC₅₀ | half maximal inhibitory concentration | | |

### General procedure for the synthesis of triphosphate phosphoramidates

Compounds of the invention can be prepared according to General Scheme A. It may be that any hydroxy and/or amino groups on the nucleoside moiety R⁶ are protected with a protecting group that can be removed at the end of the synthesis.

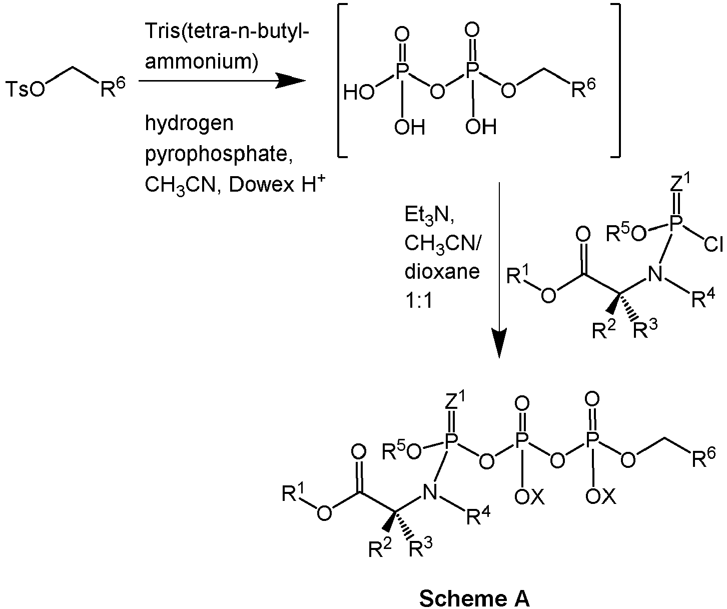

### Examples

Certain compounds of the invention can be prepared according to or analogously to **Schemes 1-6**:

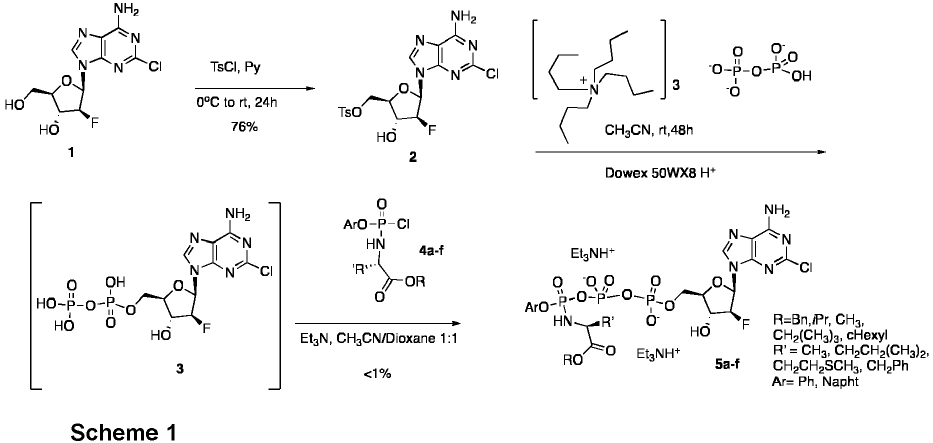

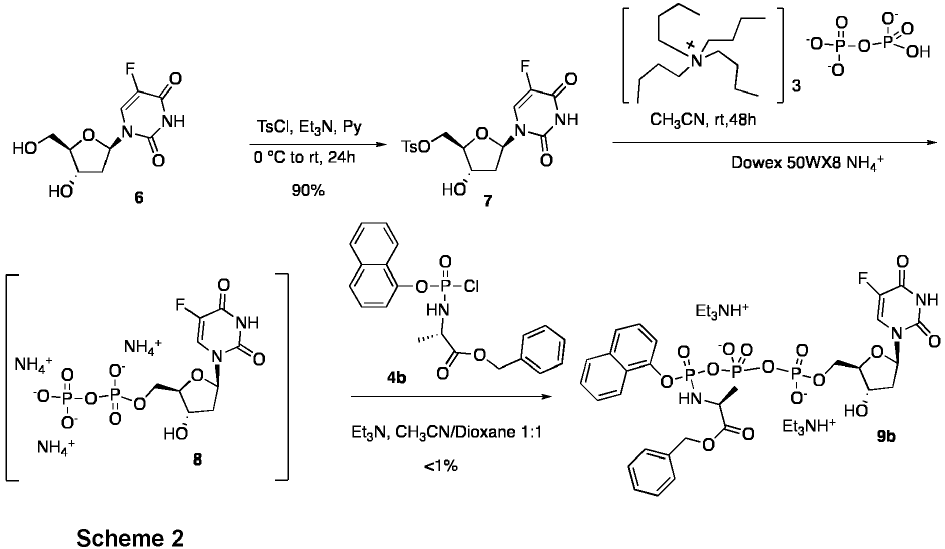

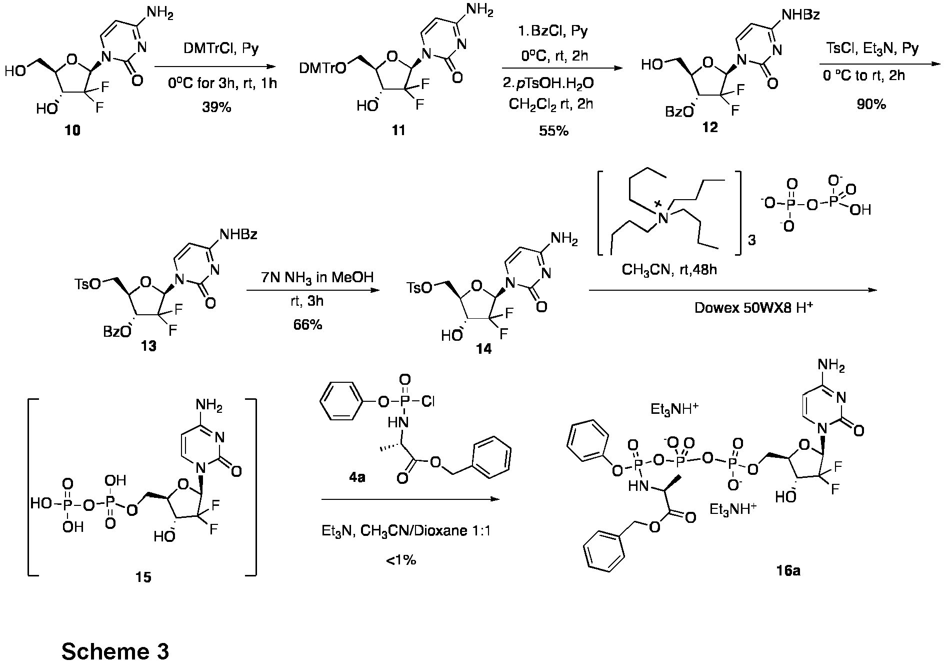

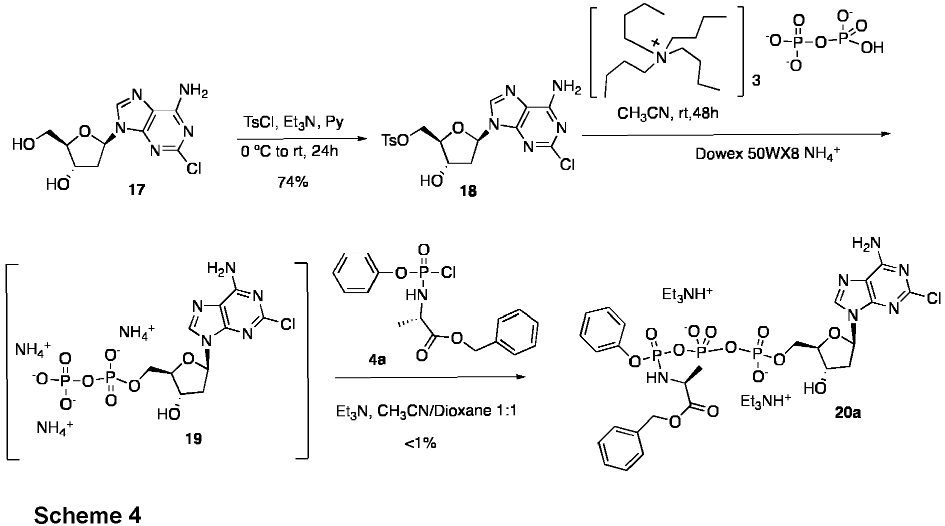

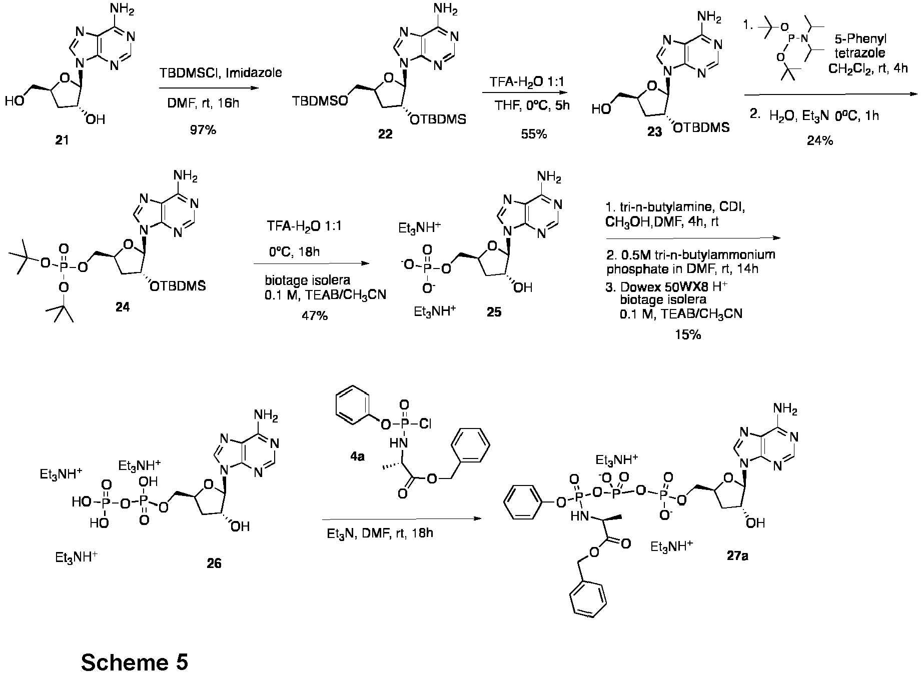

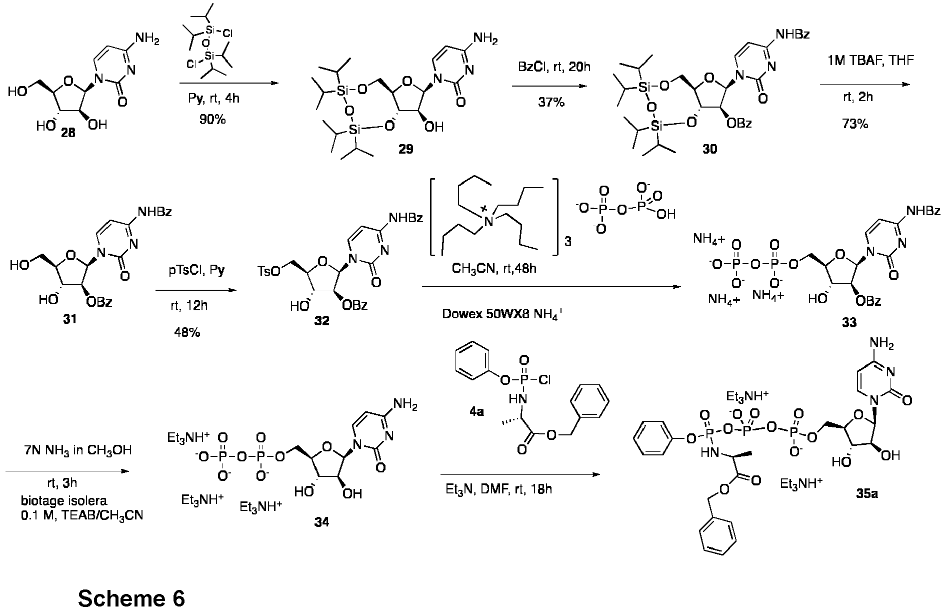

### General procedure to prepare the diphosphate and the triphosphate phosphoramidate prodrugs (5a-f, 9b, 16a and 20a)

In a 5 mL round-bottom flask Tris(tetra-*n*-butylammonium) hydrogen pyrophosphate (0.66 mmol) is added to a 1.3M solution of 5'-*para*-toluenesulphonate ester of nucleoside (0.44 mmol) in dry acetonitrile, and the mixture is stirred for 48 h at room temperature and under argon atmosphere. Upon completion, the solvent was removed under reduce pressure on a rotary evaporator and the residue was dissolved in deionized water (1 ml). (Tetra-*n-*butylammonium) cation is exchanged for proton by passing the solution through a DOWEX 50WX8 column (100-200 mesh, 12 equiv, H⁺ or NH₄⁺ form) and eluting with 3 column volumes of deionized water. The solvent was removed under vacuum and the crude product was used in the next step without further purification. A mixture of crude nucleoside 5'-diphosphate (1 mmol) in 1:1 mixture of 1,4-dioxane/acetonitrile or DMF (20 mL) was stirred at room temperature (rt) under an argon atmosphere and an appropriate phosphorochloridate **4a-f** (1.0 -1.2 eq.), dissolved in anhydrous acetonitrile or DMF, was added dropwise. The resulting reaction mixture was stirred for 18 h at rt. The crude mixture was evaporated under reduced pressure and the residue was dissolved in 0.1 M TEAB and washed with DCM. After evaporation of the water phase the crude is purified on Biotage Isolera One (C18 SNAP Ultra 30, 60, 120 g cartridge with gradient of 0.1 M TEAB in acetonitrile from 100 to 0% as an eluent in 45 min.) or by HPLC on semi-preparative column (Varian Pursuit XRs 5C18, 150 x 21.22 mm) using gradient of 0.1 M TEAB in acetonitrile from 90% to 0% in 35 min.

### (2S)-Benzyl 2-(((((((((2R, 3R,4S, 5R)-5-(6-amino-2-chloro-9H-purin-9-yl)-4-fluoro-3-hydroxytetrahydrofuran-2-yl)methoxy)(hydroxy)phosphoryl)oxy) (hydroxy)phosphoryl)oxy)(phenoxy)phosphoryl)amino)propanoate, di-triethylammonium salt (5a).

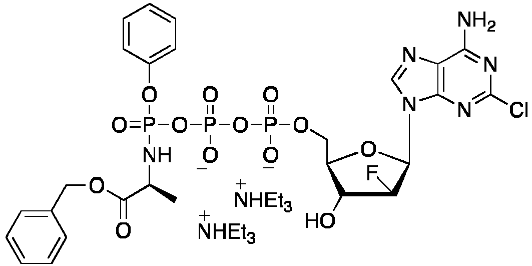

Prepared according general procedure starting from ((2*R*,3*R*,4*S*,5*R*)-5-(6-amino-2-chloro-9*H*-purin-9-yl)-4-fluoro-3-hydroxytetrahydrofuran-2-yl)methyl 4-methylbenzenesulfonate (**2**) (250 mg, 0.56 mmol), (tetrabutylammonium)hydrogen pyrophosphate (740 mg, 0.82 mmol) in CH₃CN (0.6 mL) for the first step, and then crude diphosphate (126 mg, 0.27 mmol), triethylamine (0.81 mmol, 0.11 mL) and aryloxyphosphorochloridate **4a** (0.27 mmol, 96 mg) in CH₃CN/1,4-dioxane mixture (8 mL) to obtain after purification <10mg of desired compound **5a** (<1 %).¹H NMR (500 MHz, CD₃OD): δ_{H} 8.21 (d, *J*_{HF} = 1.9Hz, 1H, *H*-8), 7.22 - 7.13 (m, 10H, Ar-*H*), 6.29 (dd, *J* = 4.3 *J*_{H-F} = 15.2, 1H, *H*-1'), 6.28 (dd, *J* = 4.3 *J*_{H-F} = 14.85 Hz, 1H, *H*-1'), 5.09 - 4.97 (m, 3H, *H*-2', OC*H*₂Ph), 4.52 (ddd, *J* = 3.7 and 4.7 Hz, *J*_{H-F} = 18.3, 1H, *H*-3'), 4.26 - 4.14 (m, 3H, *H*-5') 4.07 - 4.00 (m, *H*-4' and -C*H*NH), 3.00 (q, 12H, -NC*H*₂CH₃), 1.34 (d, *J* = 7.5 Hz, 1.5H, C*H*₃), 1.23 (d, *J* = 7.6Hz, 1.5H, C*H*₃*),* 1.17 (t, *J* = 7.3 Hz, 18H, -NCH₂C*H*₃).³¹P NMR (202 MHz, CD₃OD): δ_{P} - 7.55 (d, *J*_{P-P}= 17.8 Hz, 0.5P), - 8.16 (d, *J*_{P-P} = 17.8, 0.5P), -11.64 (d, *J*_{P-P}= 21.00 Hz, 1P), - 24.31- (-24.50) (m, 1P). ¹⁹F NMR (470 MHz, CD₃OD): δ_{F} -199.75, -199.78. Reverse HPLC, eluting with a gradient of CH₃CN in 0.1M TEAB buffer pH = 7.4 from 10/90 to 100/0 in 30 min, 1mL/min, λ = 270 nm, with t_{R} = 14.17min

### (2S)-Benzyl 2-(((((((((2R,3R,4S,5R)-5-(6-amino-2-chloro-9H-purin-9-yl)-4-fluoro-3-hydroxytetrahydrofuran-2-yl)methoxy)(hydroxy)phosphoryl)oxy)(hydroxy) phosphoryl)oxy)(naphthalen-1-yloxy)phosphoryl)amino)propanoate di-triethylammonium salt (5b).

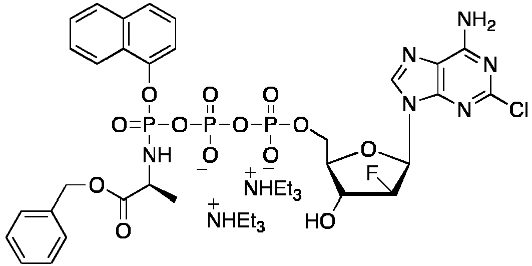

Prepared according general procedure starting from ((2*R*,3*R*,4*S*,5*R*)-5-(6-amino-2-chloro-9*H*-purin-9-yl)-4-fluoro-3-hydroxytetrahydrofuran-2-yl)methyl 4-methylbenzenesulfonate (**2**) (315 mg, 0.69 mmol), (tetrabutylammonium)hydrogen pyrophosphate (932 mg, 1.04 mmol) in CH₃CN (0.75 mL) for the first step, and then crude diphosphate (330 mg, 0.43 mmol), triethylamine (1.3 mmol, 0.2 mL) and aryloxyphosphorochloridate **4b** (0.52 mmol, 210 mg) in DMF (7 mL) to obtain after purification <15mg of desired compound **5b** (<1%).¹H NMR (500 MHz, CD₃OD): δ_{H} 8.26 - 8.19 (m, 2H, *H*-8, Ar-*H*), 7.63 - 7.69 (m, 1H, Ar-*H*), 7.55 - 7.48 (m, 2H, Ar-*H*), 7.39 - 7.35 (m, 1H, Ar-*H*), 7.29 - 7.22 (m, 1H, Ar-*H*), 7.15 - 7.11 (m, 5H, Ar-*H*), 6.25 (dd, *J* = 4.3 *J*_{H-F} = 15.09, 1H, *H*-1'), 5.01 (dd, *J* = 3.9 Hz, 7. Hz, *J*_{H-F} = 52.1 Hz, 1H, *H*-2'), 4.90 - 4.81 (m, 2H, - OC*H*₂Ph), 4.53 - 4.48 (m, 1H, *H*-3'), 4.26 - 4.00 (m, 4H, *H*-5', *H*-4' and NHC*H*CH3), 3.04 (q, 12H, 2 x (NC*H*₂CH₃)₃), 1.33 (d, *J* = 7.5 Hz, 1.5H, C*H*₃), 1.18 (d, *J* = 7.6Hz, 1.5H, C*H*₃*),* 1.15 (t, *J* = 7.3 Hz, 18H, 2 x (NC*H*₂CH₃)₃).³¹P NMR (202 MHz, CD₃OD): δ_{P} -7.17 (d, *J*_{P-P} = 17.8 Hz, 0.5P), - 7.92 (d, *J*_{P-P} = 17.8 Hz, 0.5P), -11.40 (d, *J*_{P-P} = 19.8 Hz, 0.5P), -11.44 (d, *J*_{P-P} = 20.2 Hz, 0.5P), -23.89 - (-24.16) (m, 1P). ¹⁹F NMR (470 MHz, CD₃OD): δ_{F} -199.83, -199.85. Reverse HPLC, eluting with a gradient of CH₃CN in 0.1M TEAB buffer pH= 7.4 from 10/90 to 100/0 in 30 min, 1mL/min, λ = 270 nm, with t_{R} = 13.55 min.

### (2S)-Isopropyl2-(((((((((2R,3R,4S,5R)-5-(6-amino-2-chloro-9H-purin-9-yl)-4-fluoro-3-hydroxytetrahydrofuran-2-yl)methoxy)(hydroxy)phosphoryl)oxy)(hydroxy) phosphoryl)oxy)(phenoxy)phosphoryl)amino)propanoate di-triethylammonium salt (5c).

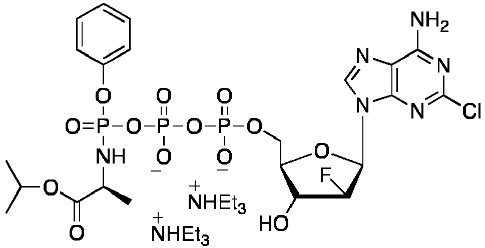

Prepared according general procedure starting from ((2*R*,3*R*,4*S*,5*R*)-5-(6-amino-2-chloro-9*H*-purin-9-yl)-4-fluoro-3-hydroxytetrahydrofuran-2-yl)methyl 4-methylbenzenesulfonate (**2**) (315 mg, 0.7 mmol), (tetrabutylammonium)hydrogen pyrophosphate (932 mg, 1.04 mmol) in CH₃CN (0.75 mL) for the first step, and then crude diphosphate (330 mg, 0.45 mmol), triethylamine (1.3 mmol, 0.2 mL) and aryloxyphosphorochloridate **4c** (0.68 mmol, 158 mg) in DMF (7 mL) to obtain after purification <10mg of desired compound **5c** (<1%). ¹H NMR (500 MHz, CD₃OD): δ_{H} 8.23 (d, *J*_{HF}= 1.9Hz, 1H, *H*-8), 7.26 - 7.00 (m, 5H, Ar-*H*), 6.31 (dd, *J* = 4.3 Hz, *J*_{H-F} = 15.2 Hz, 1H, *H-*1'), 5.05 (dd, *J* = 3.9 Hz, 4.4 Hz, *J*_{H-F} = 52.1 Hz, 1H, *H*-2'), 4.86 - 4.80 (m, 1H, OC*H*(CH₃)₂), 4.57 - 4.51 (m, 1H, *H*-3'), 4.25 - 4.15 (m, 3H, *H*-5') 4.07 - 4.04 (m, 1H, *H*-4'), 3.97 - 3.89 (m, 1H, NHC*H*CH₃), 2.84 (q, 12H, 2 x (NC*H*₂CH₃)₃), 1.33 (d, *J* = 7.5 Hz, 1.5H, C*H*₃), 1.22 (d, *J* = 7.6 Hz, 1.5H, C*H*₃), 1.12 - 1.07 (m, 24H, NHCH(C*H*₃)₂, 2 x (NCH₂C*H*₃)₃).³¹P NMR (202 MHz, CD₃OD): δ_{P} -7.10 (d, *J*_{P-P}= 17.8 Hz, 0.5P), - 7.6 (d, *J*_{P-P}= 17.8 Hz, 0.5P), -11.41 (d, *J*_{P}. _{P} = 20.85 Hz, 1P), -11.45 (d, *J*_{P-P} = 20.85 Hz, 1P), -23.67- (-23.65) (m, 1P).

### (2S)-Methyl 2-(((((((((2R,3R,4S,5R)-5-(6-amino-2-chloro-9H-purin-9-yl)-4-fluoro-3-hydroxytetrahydrofuran-2-yl)methoxy)(hydroxy)phosphoryl)oxy)(hydroxy)phosphoryl)oxy)(phenoxy)phosphoryl) amino)-4-(methylthio)butanoate di-triethylammonium salt (5d).

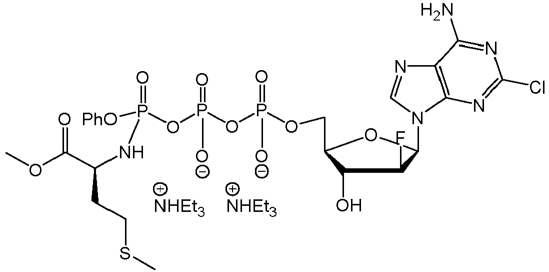

Prepared according general procedure starting from ((2R,3R,4S,5R)-5-(6-amino-2-chloro-9H-purin-9-yl)-4-fluoro-3-hydroxytetrahydrofuran-2-yl)methyl 4-methylbenzenesulfonate (**2**) (315 mg, 0.7 mmol), (tetrabutylammonium)hydrogen pyrophosphate (932 mg, 1.05 mmol) in CH3CN (0.75 mL) for the first step, and then crude diphosphate (345 mg, 0.45 mmol), triethylamine (1.3 mmol, 0.2 mL) and aryloxyphosphorochloridate **4d** (0.54 mmol, 182 mg) in DMF (7 mL) to obtain after purification <10mg of desired compound **5d** (<1%).¹H NMR (500 MHz, CD₃OD): δ_{H} 8.34 (d, *J*_{HF} = 2.0 Hz, 1H, *H*-8), 7.35 - 7.32 (m, 2H, Ar-*H*), 7.28 - 7.24 (m, 1H, Ar-*H*), 7.21 - 7.19 (m, 1H, Ar-*H*), 7.17 - 7.14 (m, 1H, Ar-*H*), 6.42 (dd, *J* = 4.0 Hz, *J*_{H-F} = 15.0 Hz, 1H, *H*-1'), 5.17 (dt, *J* = 3.0 Hz, *J*_{H-F} = 52.0 Hz, 1H, *H*-2'), 4.64 (dt, *J* = 3.5 Hz, *J*_{H-F} = 18.0 Hz, 1H, *H*-3'), 4.39 - 4.28 (m, 2H, *H*-5'), 4.20 - 4.12 (m, *H*-4', NHC*H*), 3.64, 3.63 (2 x s, 3H, OCH₃), 3.00 (q, 12H, 2 x (NC*H*₂CH₃)₃), 2.49 - 2.38 (m, 2H, NHCHC*H*₂CH₂SCH₃), 2.05 - 1.81 (m, 5H, NHCHCH₂C*H*₂SCH₃), 1.17 (t, *J* = 7.3 Hz, 18H, 2 x (NCH₂C*H*₃)₃). ³¹P NMR (202 MHz, CD3OD): δ_{P} -7.65 (d, J_{P-P} = 19.40 Hz, 1P), -11.69 (d, J_{P-P} = 20.6 Hz, 0.5P), -11.75 (d, J_{P-P} = 20.6 Hz, 0.5P), -24.44 - (-24.69) (m, 1P).

### (2S)-Cyclohexyl 2-(((((((((2R,3R,4S,5R)-5-(6-amino-2-chloro-9H-purin-9-yl)-4-fluoro-3-hydroxytetrahydrofuran-2-yl)methoxy)(hydroxy)phosphoryl)oxy)(hydroxy)phosphoryl)oxy)(phenoxy)phosphoryl) amino)-3-methylbutanoate di-triethylammonium salt (5e).

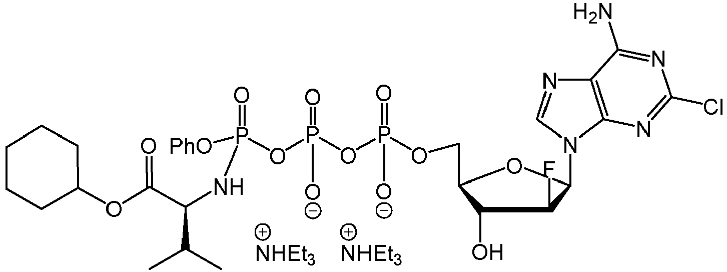

Prepared according general procedure starting from ((2R,3R,4S,5R)-5-(6-amino-2-chloro-9H-purin-9-yl)-4-fluoro-3-hydroxytetrahydrofuran-2-yl)methyl 4-methylbenzenesulfonate (**2**) (315 mg, 0.7 mmol), (tetrabutylammonium)hydrogen pyrophosphate (932 mg, 1.05 mmol) in CH₃CN (0.75 mL) for the first step, and then crude diphosphate (345 mg, 0.45 mmol), triethylamine (1.3 mmol, 0.2 mL) and aryloxyphosphorochloridate **4e** (0.54 mmol, 202 mg) in DMF (7 mL) to obtain after purification <10mg of desired compound **5e** (<1%).¹H NMR (500 MHz, CD₃OD): δ_{H} 8.19 (apparent t, *J*_{H-F} = 2.0 Hz, 1H, *H*-8), 7.21 - 7.14 (m, 3H, Ar-*H*), 7.03 - 6.96 (m, 2H, Ar-*H*), 6.41 (dd, *J* = 4.5 Hz, *J*_{H-F} = 15.0 Hz, 1H, *H*-1'), 5.15 (dt, *J* = 3.0 Hz, *J*_{H-F}= 52.0 Hz, 1H, H-2'), 4.76 - 4.57 (m, 1H, OC*H*-cycHexyl), 4.69 - 4.63 (m, 1H, *H*-3'), 4.25 - 4.12 (m, 2H, H-5'), 4.02 - 4.00 (m, 1H, *H*-4'), 3.66 (dd, *J* = 10 Hz, 5.0 Hz, 0.5H, NHC*H*CH(CH₃)₂), 3.59 (dd, *J* = 10 Hz, 5.0 Hz, 0.5H, NHC*H*CH(CH₃)₂), 3.00 (q, 12H, 2 x (NC*H*₂CH₃)₃), 1.94 - 1.82 (m, 1H, NHCHC*H*(CH₃)₂), 1.71 - 1.67 (m, 1H, C*H*₂ₐ-cyclohexyl), 1.63 - 1.54 (m, 3H, 1H, CH_{2b}-cyclohexyl), 1.40 - 1.19 (m, 6H, 3 x C*H*₂-cyclohexyl), 0.86 (apparent t, *J* = 7.0 Hz, 3H, NHCHCH(C*H*₃)₂), 0.75 (d, *J* = 7.0 Hz, 1.5H, NHCHCH(C*H*₃)₂), 0.71 (d, *J* = 7.0 Hz, 1.5H, NHCHCH(C*H*₃)₂), 1.17 (t, *J* = 7.3 Hz, 18H, 2 x (NCH₂C*H*₃)₃). ³¹P NMR (202 MHz, CD₃OD): δ_{P} -6.90 (d, *J*_{P-P}= 19.60 Hz, 0.5P), -7.17 (d, *J*_{P-P}= 19.60 Hz, 0.5P), -11.66 (d, *J*_{P-P}= 21.0 Hz, 0.5P), -11.68 (d, *J*_{P-P} = 21.0 Hz, 0.5P), -24.32 - (-24.58) (m, 1P).

### (2S)-Neopentyl 2-(((((((((2R,3R,4S,5R)-5-(6-amino-2-chloro-9H-purin-9-yl)-4-fluoro-3-hydroxytetrahydrofuran-2-yl)methoxy)(hydroxy)phosphoryl)oxy)(hydroxy)phosphoryl)oxy)(phenoxy)phosphoryl) amino)-3-phenylpropanoate di-triethylammonium salt (5f).

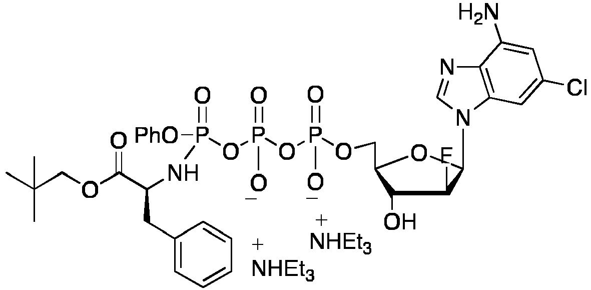

Prepared according general procedure starting from ((2R,3R,4S,5R)-5-(6-amino-2-chloro-9H-purin-9-yl)-4-fluoro-3-hydroxytetrahydrofuran-2-yl)methyl 4-methylbenzenesulfonate (**2**) (315 mg, 0.7 mmol), (tetrabutylammonium)hydrogen pyrophosphate (932 mg, 1.05 mmol) in CH₃CN (0.75 mL) for the first step, and then crude diphosphate (345 mg, 0.45 mmol), triethylamine (1.3 mmol, 0.2 mL) and aryloxyphosphorochloridate **4f** (0.54 mmol, 221 mg) in DMF (7 mL) to obtain after purification <10mg of desired compound **5f** (<1%).¹H NMR (500 MHz, CD₃OD): δ_{H} 8.33 (d *J*_{H-F} = 2.0 Hz, 0.5H, *H*-8), 8.32 (d *J*_{H-F} = 2.0 Hz, 0.5H, *H*-8), 7.35 - 7.13 (m, 10H, Ar-*H*), 6.40 (dd, *J* = 4.3 *J*_{H-F}= 14.85 Hz, 1H, *H*-1'), 5.16 (dt, *J* = 3.5Hz, *J*_{H-F}= 52.1 Hz, 1H, *H*-2'), 4.67 - 4.60 (m, 1H, *H*-3'), 4.37 - 4.09 (m, 5H, *H*-5', *H*-4', OC*H*₂(CH₃)₃), 3.30 - 3.21 (m, 1H, NHC*H*₂Ph), 3.00 (q, 12H, 2 x (NC*H*₂CH₃)₃), 1.17 (m, 27H, OCH₂(C*H*₃)₃, 2 x (NCH₂C*H*₃)₃). ³¹P NMR (202 MHz, CD₃OD): δ_{P} -5.50 (d, *J*_{P-P}= 19.60 Hz, 0.5P), -5.48 (d, *J*_{P-P} = 19.60 Hz, 0.5P), -11.67 (d, *J*_{P-P} = 21.0 Hz, 0.5P), -11.66 (d, *J*_{P-P} = 21.0 Hz, 0.5P), -24.28 - (-24.50) (m, 1P).

### (2S)-Benzyl 2-(((((((((2R,3S,5R)-5-(5-fluoro-2,4-dioxo-3,4-dihydropyrimidin-1 (2H)-yl)-3-hydroxytetrahydrofuran-2-yl)methoxy)(hydroxy)phosphoryl)oxy)(hydroxy)phosphoryl)oxy)(naphthalen-1-yloxy)phosphoryl)amino)propanoate di-triethylammonium salt (9b).

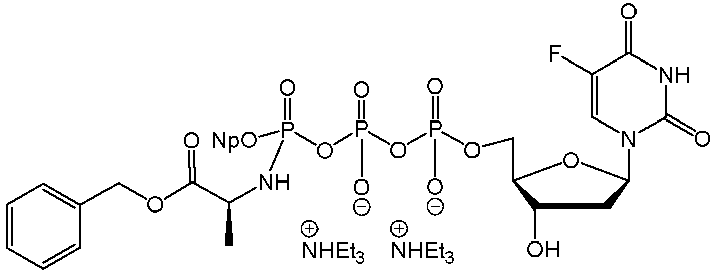

Prepared according general procedure starting from ((2*R*,3*S*,5*R*)-5-(5-fluoro-2,4-dioxo-3,4-dihydropyrimidin-1(2*H*)-yl)-3-hydroxytetrahydrofuran-2-yl)methyl 4-methylbenzenesulfonate (**7**) (1g, 2.50 mmol), (tetrabutylammonium)hydrogen pyrophosphate (2.7 g, 3 mmol) in CH₃CN (2.7 mL) for the first step, and then crude diphosphate (600 mg, 1.3 mmol), triethylamine (3.9 mmol, 7 mL) and aryloxyphosphorochloridate **6b** (1.3 mmol, 513 mg) in DMF (10 mL) to obtain after purification <10 mg of desired compound **9b** (<1%). ¹H NMR (500 MHz, CD₃OD): δ_{H} 8.24 - 8.22 (m, 1H, Ar-*H*), 7.92 (d, *J*_{H-F} = 6.7 Hz, 1H, *H*-6), 7.75 - 7.72 (m, 1H, Ar-*H*), 7.56 - 7.50 (m, 2H, Ar-*H*), 7.41 - 7.38 (m, 1H, Ar-*H*), 7.31 - 7.24 (m, 1H, Ar-*H*), 7.17 - 7.10 (m, 5H, Ar-*H*), 6.13 - 6.08 (m, 1H, *H*-1'), 4.92 - 4.83 (m, 2H, - OC*H*₂Ph), 4.45 - 4.42 (m, 1H, *H*-3'), 4.16 - 4.02 (m, 3H, *H*-5' NHC*H*CH₃), 3.89 - 3.87 (m, 1H, *H*-4'), 3.04 (q, 6H, 2 x (NC*H*₂CH₃)₃), 1.33 (d, *J* = 7.5 Hz, 1.5H, C*H*₃), 1.19 (d, *J* = 7.6 Hz, 1.5H, C*H*₃), 1.18 (t, *J* = 7.3 Hz, 9H, 2 x (NCH₂C*H*₃)₃).³¹P NMR (202 MHz, CD₃OD): δ_{P} -6.93 (d, *J*_{P-P} = 17.8 Hz, 0.5P), - 7.59 (d, *J*_{P-P} = 17.8 Hz, 0.5P), -11.22 (d, *J*_{P-P} = 19.3 Hz, 0.5P), - 11.33 (d, *J*_{P-P} = 21.2 Hz, 0.5P), -23.37 - (-23.63) (m, 1P). ¹⁹F NMR (470 MHz, CD₃OD): δ_{F} - 167.28, -167.29. Reverse HPLC, eluting with a gradient of CH₃CN in 0.1M TEAB buffer pH= 7.4 from 10/90 to 100/0 in 30 min, 1mL/min, λ = 270 nm, with t_{R} = 12.97 min.

### (2S)-Benzyl2-(((((((((2R,3R,5R)-5-(4-amino-2-oxopyrimidin-1(2H)-yl)-4,4-difluoro-3-hydroxytetrahydrofuran-2-yl)methoxy)(hydroxy)phosphoryl)oxy)(hydroxy)phosphoryl)oxy)(phenoxy)phosphoryl) amino)propanoate di-triethylammonium salt (16a).

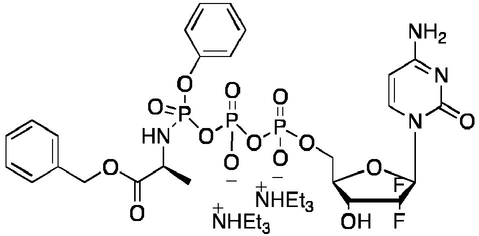

Prepared according general procedure starting from ((2*R*,3*R*,5*R*)-5-(4-amino-2-oxopyrimidin-1(2*H*)-yl)-4,4-difluoro-3-hydroxytetrahydrofuran-2-yl)methyl-4-methylbenzenesulfonate (**14**) (95 mg, 0.23 mmol), (tetrabutylammonium)hydrogen pyrophosphate (308 mg, 0.34 mmol) in CH₃CN (0.3 mL) for the first step and then crude diphosphate (96 mg, 0.23 mmol), triethylamine (0.66 mmol, 0.9 mL) and aryloxyphosphorochloridate **4a** (0.23 mmol, 92 mg) in DMF (3 mL) to obtain after purification <10 mg of desired compound **16a** (<1%). ¹H NMR (500 MHz, CD₃OD): δ_{H} 7.79 (d, *J=* 7.5 Hz, 0.5H, *H*-6), 7.78 (d, *J* = 7.5 Hz, 0.5H, *H*-6), 7.23 - 7.16 (m, 10H, Ar-*H*), 6.13 - 6.07 (m, 1H, *H*-1'), 5.89 (d, *J* = 7.5 Hz, 0.5H, *H*-5), 5.85 (d, *J* = 7.5 Hz, 0.5H, *H*-5), 5.04 - 4.96 (m, 2H, OC*H*₂Ph), 4.34-4.18 (m, 3H, *H*-5', *H*-3'), 4.06 - 4.00 (m, 1H, C*H*NHCH₃), 3.87 - 3.84 (m, 1H, *H*-4'), 3.07 (q, 6H, 2 x (NC*H*₂CH₃)₃), 1.34 (d, *J* = 7.6 Hz, 1.5H, C*H*₃*),* 1.24 (d, *J* = 7.6 Hz, 1.5H, C*H*₃*),* 1.18 (t, *J* = 7.3 Hz, 9H, 2 x (NCH₂C*H*₃)₃).³¹P NMR (202 MHz, CD₃OD): δ_{P} -7.16 (d, *J*_{P-P}= 17.5 Hz, 0.5P), - 7.64 (d, *J*_{P-P}= 17.5 Hz, 0.5P), -11.22 (d, *J*_{P-P}= 18.34 Hz, 0.5P), -11.36 (d, *J*_{P-P} = 18.34 Hz, 0.5P), -23.40 - (-23.59) (m, 1P). ¹⁹F NMR (470 MHz, CD₃OD): δ_{F} -119.6 (d, *J*_{F-F}= 240.21 Hz, 0.5F), -119.2 (d, *J*_{F-F} = 240 Hz, 0.5F), -120.31 (d, *J*_{F-F}= 241.74 Hz, F). Reverse HPLC, eluting with a gradient of CH₃CN in 0.1M TEAB buffer pH= 7.4 from 10/90 to 100/0 in 30 min, 1mL/min, λ = 263 nm, with t_{R} = 12.10 min (88%).

### (2S)-Benzyl 2-(((((((((2R, 3S, 5R)-5-(6-amino-2-chloro-9H-purin-9-yl)-3-hydroxytetrahydrofuran-2-yl)methoxy)(hydroxy)phosphoryl)oxy)(hydroxy)phosphoryl)oxy)(phenoxy)phosphoryl) amino)propanoate di-triethylammonium salt (20a).

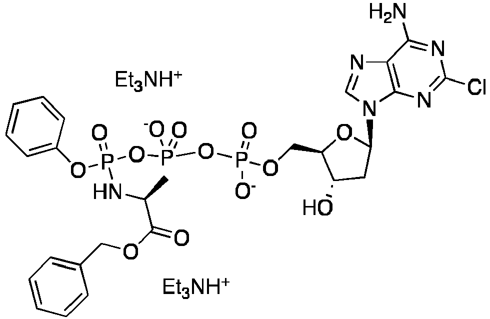

Prepared according general procedure starting from ((2*R*,3*S*,5*R*)-5-(6-amino-2-chloro-9*H*-purin-9-yl)-3-hydroxytetrahydrofuran-2-yl)methyl 4-methylbenzenesulfonate (**18**) (210 mg, 0.50 mmol), (tetrabutylammonium)hydrogen pyrophosphate (700 mg, 0.77 mmol) in CH₃CN (0.8 mL) for the first step, and then crude diphosphate (164 mg, 0.33 mmol), triethylamine (0.99 mmol, 0.13 mL) and aryloxyphosphorochloridate **4a** (0.39 mmol, 140 mg) in DMF (8 mL) to obtain after purification <10mg of desired compound **20a** (<1%).¹H NMR (500 MHz, CD₃OD): δ_{H} 8.48 (s, 1H, *H*-8), 7.33 - 7.27 (m, 9H, Ar-*H*), 7.14 - 7.11 (m, 1H, Ar-*H*), 6.41 (t, *J* = 6.6 Hz, 1H, *H*-1'), 6.28 (t, *J* = 6.6 Hz, 1H, *H*-1'), 5.11 (s, 1H, OC*H*₂Ph), 5.10 (s, 1H, OC*H*₂Ph), 4.52 (m, 1H, *H*-3'), 4.26 - 4.14 (m, 4H, *H*-5', *H*-4', NHC*H*CH₃), 2.79 (q, 12H, 2 x (NC*H*₂CH₃)₃), 2.75-2.62 (m, 1H, *H*-2'), 2.46 - 2.09 (m, 1H, H-2'), 1.46 (d, *J* = 7.5 Hz, 1.5H, C*H*₃*),* 1.35 (d, *J* = 7.6 Hz, 1.5H, C*H*₃*),* 1.14 (t, *J* = 7.3 Hz, 18H, 2 x (NCH₂C*H*₃)₃).³¹P NMR (202 MHz, CD₃OD): δ_{P} -7.57 (d, *J*_{P-P}= 18.7 Hz, 0.5P), - 8.17 (d, *J*_{P-P}= 18.2 Hz, 0.5P), -11.79 (d, *J*_{P-P} = 20.6 Hz, 0.5P), -11.83 (d, *J*_{P-P} = 20.2 Hz, 0.5P), -24.31 - (-24.50) (m, 1P).

### General procedure to prepare (2S)-benzyl 2-(((((((((2S,4R,5R)-5-(6-amino-9H-purin-9-yl)-4-hydroxytetrahydrofuran-2-yl)methoxy)(hydroxy)phosphoryl)oxy)(hydroxy)phosphoryl)oxy)(phenoxy)phosphoryl) amino)propanoate di-triethylammonium salt (27a).

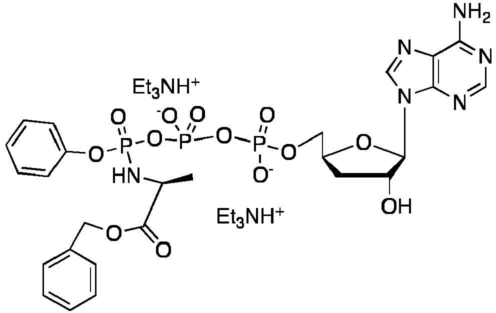

### 9-((2R,3R,5S)-3-((tert-butyldimethylsilyl)oxy)-5-(((tert-butyldimethylsilyl)oxy)methyl)tetrahydrofuran-2-yl)-9H-purin-6-amine (22).

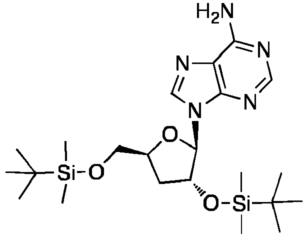

3'-Deoxyadenosine (**21**) (1.13 g, 4.5 mmol), was dissolved in DMF (50 mL) and TBDMSCI (2.03 g, 13.5 mmol) and imidazole (1.84 g, 27 mmol) were added to the flask. The mixture was stirred for 16 hours at rt. The reaction mixture was diluted with CHCl₃ (20 mL) and the organics were washed with NH₄Cl (saturated solution, 20 mL x 3). The organics were dried over Na₂SO₄, filtered and evaporated to afford **22** as a sticky solid (1.56 g, 72%). ¹H NMR (500 MHz, CDCl₃) δ_{H} 8.36 (s, 1H, *H*-8), 8.34 (s, 1H, *H*-2), 6.03 (d, *J* = 1.2 Hz, 1H, *H*-1'), 5.54 (br s, 2H, N*H*₂), 4.66 - 4.63 (m, 1H, *H*-2'), 4.61 - 4.55 (m, 1H, *H*-4'), 4.14 (dd, *J* = 11.7 Hz, 2.7 Hz, 1H, *H*-5'), 3.80 (dd, *J* = 11.7 Hz, 2.7 Hz, 1H, *H*-5'), 2.32 - 2.25 (m, 1H, *H-*3'), 1.91 - 1.85 (m, 1H, *H*-3'), 0.97 (s, 9H, *tert*-But), 0.92 (s, 9H, *tert*-But), 0.17 (s, 3H, C*H*₃), 0.16 (s, 3H, C*H*₃), 0.15 (s, 3H, C*H*₃), 0.10 (s, 3H, C*H*₃).

### ((2S,4R,5R)-5-(6-amino-9H-purin-9-yl)-4-((tert-butyldimethylsilyl)oxy)tetrahydrofuran-2-yl)methyl di-tert-butyl phosphate (23).

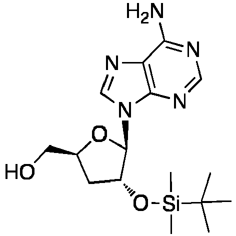

Compound **22** (1.56 g, 3.25 mmol) was dissolved in THF (2 mL) and cooled down to 0 °C. A solution of TFA in water (1 mL, 1/1 v/v) was added dropwise and the mixture was stirred for 5 hours at 0°C. The solution was evaporated under *vacuum* and the crude purified via Biotage Isolera One (30g ZIP cartridge KP SIL, 60 mL/min, gradient eluent system 2-20% CH₃OH/CH₂Cl₂ 10CV, 20% 5CV) to yield **23** as a white foam (1.16 g, 98%). ¹H NMR (500 MHz, CDCl₃) δ_{H} 8.45 (s, 1H, *H*-8), 7.97 (s, 1H, *H*-2), 6.29 (br s, 2H, N*H*₂), 5.74 (d, *J* = 6.0 Hz, 1H, *H*-1'), 5.19 - 5.12 (m, 1H, *H*-2'), 4.65-4.60 (m, 1H, *H*-4'), 4.11 (dd, *J* = 13.0 Hz, 1.5 Hz, 1H, *H*-5'), 3.67 (dd, *J* = 12.5 Hz, 1.5 Hz, 1H, *H*-5'), 2.72 - 2.62 (m, 1H, *H*-3'), 2.37 - 2.26 (m, 1H, *H*-3'), 1.48 (d, *J* = 7.0 Hz, 1H, O*H*-5'), 0.91 (s, 9H, *tert*-But), 0.00 (s, 3H, C*H*₃), - 0.12 (s, 3H, C*H*₃).

### ((2S,4R,5R)-5-(6-amino-9H-purin-9-yl)-4-((tert-butyldimethylsilyl)oxy)tetrahydrofuran-2-yl)methyl di-tert-butyl phosphate (24).

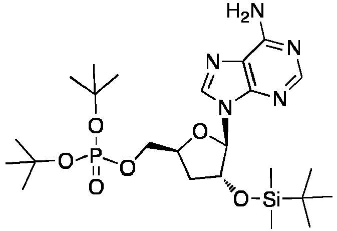

To a solution of compound **23** (0.960 g, 2.63 mmol) in dry CH₂Cl₂ (35 mL) under argon 5-phenyl-1H-tetrazole (0.770 g, 5.26 mmol) was added followed by the addition dropwise of *N,N*-di-tertbutyl disopropyl phosphoramidite (1.25 mL, 3.95 mmol). After stirring at rt for 4 h the mixture is cooled down to 0 °C and Et₃N (1.37 mL, 9.86 mmol) is added dropwise followed by dropwise addition of 30% H₂O₂ (1.24 mL, 14.24 mmol). After 2 hour stirring at the same temperature a saturated solution of NaHCO₃ is addded ad after 20 minutes stirring the phases were separated. The organic phase was dried with Na₂SO₄ , filtered and evaporated to obtain a crude product that was purified by isolera Biotage Isolera One (SNAP ultra 100 gradient eluent system 5/85/10 CH₃OH/acetone /CH₂Cl₂ from 0 to 8% of methanol) to yield **24** as a white foam (0.340 g, 24%). ¹H NMR (500 MHz, CD₃OD) δ_{H} 8.34 (s, 1H, *H*-8), 8.24 (s, 1H, *H*-2), 6.00 (d, *J* = 2.5 Hz, 1H, *H*-1'), 4.88 - 4.82 (m, 1H, *H*-2'), 4.67 - 4.66 (m, 1H, *H*-4'), 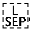 4.37 - 4.29 (m, 1H, C*H*₂ₐ-5'), 4.19 - 4.14 (m, 1H, C*H*_{2b}-5'), 2.39 - 234 (m, 1H, *H*-3'), 2.14 - 2.10 (m, 1H, *H*-3'), 1.49 (s, 9H, OC(C*H*₃)₃), 1.48 (s, 9H, OC(C*H*₃)₃), 0.86 (s, 9H, SiC(C*H*₃)₃), 0.00 (s, 3H, C*H*₃), -0.12 (s, 3H, C*H*₃). ³¹P NMR (202 MHz, CD₃OD): -10.34 (1P)

### Bis(triethylammonium) mono(((2S,4R,5R)-5-(6-amino-9H-purin-9-yl)-4-hydroxytetrahydrofuran-2-yl)methyl hydrogenphosphate) (25).

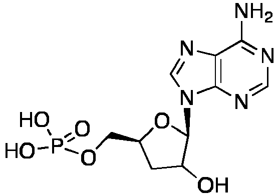

To a solution of compound **24** (0.340 g, 1.03 mmol) in H₂O (12 mL) at 0°C was added dropwise of TFA (6 mL). After stirring at 0 °C for 18 h the solvent is evaporated and the residue is taken up with water (10 ml), washed with EtOAc (2 x 5mL) anf the soleven removed under pressure to obtain **25** as white solid (0.200 g, 99%) ¹H NMR (500 MHz, CD₃OD) δ_{H} 8.56 (s, 1H, *H*-8), 8.40 (s, 1H, *H*-2), 6.10 (d, *J* = 2.5 Hz, 1H, *H*-1'), 4.78 - 4.69 (m, 2H, *H*-2', *H*-4'), 4.38 - 4.34 (m, 1H, C*H*₂ₐ-5'), 4.18 - 4.12 (m, 1H, C*H*_{2b}-5'), 2.43 - 2.39 (m, 1H, *H*-3'), 2.15-2.11 (m, 1H, *H*-3'), ³¹P NMR (202 MHz, CD₃OD): 0.046 (1P).

### Triethylammonium ((2S,4R,5R)-5-(6-amino-9H-purin-9-yl)-4-hydroxytetrahydrofuran-2-yl)methyl diphosphate (26).

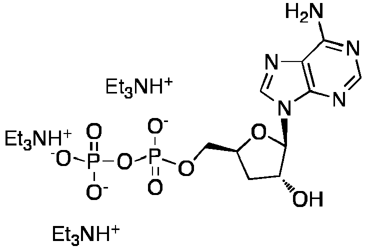

To a solution of compound **25** (0.100 g, 0.30 mmol) in dry DMF (2 mL) under argon tributylamine (0.072 mL, 0.30 mmol) was added and the resulting suspension was stirred for 30 min at rt. The solvent was removed under pressure and the residue was dissolved in DMF (3 mL). To this solution CDI (0.243, 1.5 mmol) was added and the reaction was stirred for 4 h at rt, followed by addition of MeOH (0.097 mL, 2.4 mmol) and 20 min of additional stirring. To this solution 0.5 M tris-n-butylammonium phosphate in DMF (12 mL, 6 mmol) was added and the reaction stirred overnight under argon at rt. The solvent was removed under vacuum to obtain a residue, which was re-dissolved in H₂O and washed with CH₂Cl₂. The solvent was removed and the residue was dissolved in deionized water (1 ml). (Tris-n-butylammonium) cation is exchanged for proton by passing the solution through a DOWEX 50WX8 column (100-200 mesh, 12 equiv, H⁺ form) and eluting with 3 column volumes of deionized water. The solvent was removed under vacuum and the crude product was used in the next step without further purification purified by isolera Biotage Isolera One (C18 SNAP Ultra 120g cartridge with gradient of 1 M TEAB in acetonitrile from 100 to 0% as an eluent in 40 min) to yield **26** as a white foam (0.030 g, 14%). ¹H NMR (500 MHz, CD₃OD) δ_{H} 8.25 (s, 1H, *H*-8), 8.21 (s, 1H, *H*-2), 6.02 (d, *J* = 2.5 Hz, 1H, *H*-1'), 4.71 - 4.69 (m, 1H, *H*-2'), 4.66 - 4.64 (m, 1H, *H*-4'), 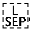 4.34 - 4.29 (m, 1H, C*H*₂ₐ-5'), 4.23 - 4.18 (m, 1H, C*H*_{2b}-5'), 3.00 (q, *J* = 7.5 Hz, 18H, 3 x (NC*H*₂CH₃)₃), 2.49 - 244 (m, 1H, *H*-3'), 2.17 - 2.12 (m, 1H, *H*-3'), 1.23 (t, *J* = 7.5 Hz, 27H, 3 x (NCH₂C*H*₃)₃). ³¹P NMR (202 MHz, CD₃OD): -9.63 (d, *J* = 22.0 Hz, 1P), -10.81 (d, *J* = 22.0 Hz, 1P)

### (2S)-Benzyl 2-(((((((((2S,4R,5R)-5-(6-amino-9H-purin-9-yl)-4-hydroxytetrahydrofuran-2-yl)methoxy)(hydroxy)phosphoryl)oxy)(hydroxy)phosphoryl)oxy)(phenoxy)phosphoryl) amino)propanoate di-triethylammonium salt (27a).

To a mixture of **26** (30 mg, 0.04 mmol) in DMF (1.5 mL) a solution of the phosphorochloridate **4a** (22 mg, 0.006 mmol, 1.5 eq) in DMF (0.5 mL) was added, followed by triethylamine (0.02 mL, 0.12 mmol, 3 eq). After stirring the resulting mixture overnight, the solvent is removed under vacuum and the residue is taken up with H₂O and washed with dichloromethane. The aqueous phase is reduced under pressure and the residue is purified with Biotage Isolera reverse phase to obtain **27a** (6 mg, 15%). ¹H NMR (500 MHz, CD₃OD): δ_{H} 8.39 (s, 1H, *H*-8), 8.08 (s, 1H, *H*-2), 6.89 (d, *J* = 3.5 Hz, 1H, *H*-1'), 5.88 (d, *J* = 3.5 Hz, 1H, *H*-1'), 7.35 - 7.16 (m, 10H, Ar-*H*), 4.99 - 4.97 (m, 2H, OC*H*₂Ph), 4.55 - 4.48 (m, 2H, *H*-2', C*H*₂ₐ-5'), 4.23 - 4.18 (m, 1H, C*H*₂ₐ-5'), 4.12 - 4.00 (m, 2H, *H*-4', NHC*H*CH₃), 3.00 (q, 12H, 2 x (NC*H*₂CH₃)₃), 2.34 - 2.22 (m, 2H, C*H*₂ₐ-3'), 2.01 - 1.99 (m, 2H, C*H*₂ₐ-3'), 1.35 (d, *J* = 7.5 Hz, 1.5H, NHCHC*H*₃), 1.22 (d, *J* = 7.6 Hz, 1.5H, NHCHC*H*₃), 1.17 (t, J = 7.3 Hz, 18H, 2 x (NCH₂C*H*₃)₃). ³¹P NMR (202 MHz, CD₃OD): δ_{P} -7.46 (d, *J*_{P-P}= 20.2 Hz, 0.5P), -8.12 (d, *J*_{P-P}= 20.2, 0.5P), -11.48 (d, *J*_{P-P} = 21.3 Hz, 1P), - 24.16 - (-24.35) (m, 1P).

### General procedure to prepare (2S)-Benzyl 2-(((((((((2R,3S,4S,5R)-5-(4-amino-2-oxopyrimidin-1 (2H)-yl)-3,4-dihydroxytetrahydrofuran-2-yl)methoxy)(hydroxy)phosphoryl)oxy)(hydroxy)phosphoryl)oxy)(phenoxy)phosphoryl) amino)propanoate di-triethylammonium salt (35a).

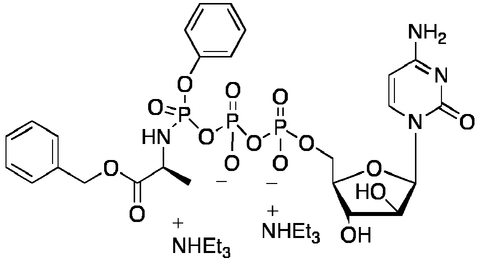

### (2R,3S,4R,5R)-2-(4-benzamido-2-oxopyrimidin-1(2H)-yl)-4-hydroxy-5-((tosyloxy)methyl)tetrahydrofuran-3-yl benzoate (32).

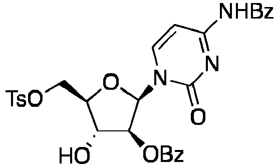

(*2R*,*3S*,*4R*,*5R*)-2-(4-benzamido-2-oxopyrimidin-1(2H)-yl)-4-hydroxy-5-(hydroxymethyl)tetrahydrofuran-3-yl benzoate (**31**) (1.12 g, 2.48 mmol), was dissolved in dry pyridine (7 mL) and p-toluensulfonyl chloride (520 mg, 2.73 mmol) was added at 0 °C. The mixture was stirred for 16 hours at rt. The reaction mixture was evaporated. The crude was dissolved in CHCl₃ (20 mL) and the organic layers were washed with water (3 x 20 mL). The organic layers were dried over Na₂SO₄, filtered and evaporated to obtain a crude residue that was purified on Biotage Isolera to yield **32** as a white solid (727 mg, 48%). ¹H NMR (500 MHz, CD₃OD): δ_{H} 7.96 - 7.94 (m, 2H, *Ar-H),* 7.83 - 7.79 (m, 4H, *Ar-H, H*-6), 7.66 - 7.61 (m, 2H, Ar-*H*), 7.56 - 7.53 (m, 2H, Ar-*H*), 7.48 - 7.42 (m, 2H, *Ar-H*), 6.41 (d, *J* = 4.5 Hz, H-5), 6.64 (dd, *J* = *2.5* Hz, 4.0 Hz, 1H, *H*-1'), 4.46 - 4.30 (m, 5H, *H*-2', *H*-5', H-4', H-3'), 2.46 (m, 3H, CH₃).

### ((2R,3R,4S,5R)-5-(4-Benzamido-2-oxopyrimidin-1(2H)-yl)-4-(benzoyloxy)-3-hydroxytetrahydrofuran-2-yl)methyl diphosphate ammonium salt (33).

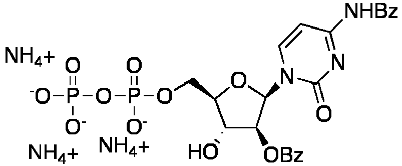

Prepared according to general procedure starting from (**32**) (720 mg, 1.19 mmol), tris(tetrabutylammonium) hydrogen pyrophosphate (1.60 g, 1.78 mmol) in CH₃CN (0.75 mL) to obtain the crude diphosphate **33** (153 mg, 0.25 mmol), which was used for the next step without further purification. ³¹P NMR (202 MHz, CD₃OD): -9.67 (d, *J* = 22.7 Hz, 1P), -10.66 (d, *J* = 22.7 Hz, 1P).

### ((2R,35,45,5R)-5-(4-amino-2-oxopyrimidin-1(2H)-yl)-3,4-dihydroxytetrahydrofuran-2-yl)methyl diphosphate triethylammonium salt (34).

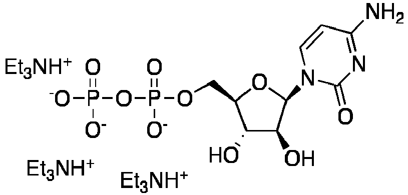

Crude diphosphate (**33**) (153 mg, 0.25 mmol) was suspended in a solution of 7N NH₃ in MeOH (0.7 ml, 5.0 mmol) and left under stirring at rt for 5 hours. After completion of reaction, the reaction mixture was evaporated and the crude residue was purified by isolera Biotage Isolera One (C18 SNAP Ultra 30g cartridge with gradient of 0.1 M TEAB in acetonitrile from 100 to 0% as an eluent in 30 min) to yield **34** as a white solid (40 mg, 32%). ¹H NMR (500 MHz, CD₃OD) δ_{H} 7.95 (d, *J=* 7.0 Hz, 1H, *H*-6), 6.22 (d, *J* = 5.5 Hz, 1H, *H*-1'), 5.91 (d, *J* = 7.3 Hz, 1H, *H*-5), 4.25 - 4.23 (m, 1H, *H*-2'), 4.21 - 4.19 (m, 1H, *H*-3'), 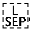 4.12 - 4.09 (m, 1H, *H*-4'), 4.04 - 4.01 (m, 2H, *H*-5'), 3.00 (q, *J* = 7.5 Hz, 18H, 3 x (NC*H*₂CH₃)₃), 1.23 (t, *J* = 7.5 Hz, 27H, 3 x (NCH₂C*H*₃)₃). ³¹P NMR (202 MHz, CD₃OD): -9.63 (d, *J* = 22.0 Hz, 1P), -10.81 (d, *J* = 22.0 Hz, 1P)

### (2S)-Benzyl 2-(((((((((2R,3S,4S,5R)-5-(4-amino-2-oxopyrimidin-1(2H)-yl)-3,4-dihydroxytetrahydrofuran-2-yl)methoxy)(hydroxy)phosphoryl)oxy)(hydroxy)phosphoryl)oxy)(phenoxy)phosphoryl) amino)propanoate di-triethylammonium salt (35a).

Prepared according general procedure step 2 starting from the crude diphosphate **34** (40 mg, 0.056 mmol), triethylamine (0.16 mmol, 0.03 mL) and aryloxyphosphorochloridate **4a** (0.056 mmol, 20 mg) in DMF (7 mL) to obtain after purification <10 mg of desired compound **35a** (<1%) ¹H NMR (500 MHz, CD₃OD): δ_{H} 7.90 (d, *J* = 7.5 Hz, 1H, *H*-6), 7.30 - 7.27 (m, 9H, Ar-*H*), 7.17 - 7.12 (m, 1H, Ar-*H*), 6.22 (d, *J* = 4.5 Hz, 1H, *H*-1'), 5.91 (d, *J* = 7.5 Hz, 0.5H, *H*-5), 5.90 (d, *J* = 7.5 Hz, 0.5H, H-5), 5.13, 5.09 (AB system apparent 2 x s, 2H, OC*H*₂Ph), 4.35 - 4.23 (m, 4H, *H*-3', *H*-4', 2 x *H*-5'), 4.19 - 4.13 (m, 1H, NHC*H*CH₃), 4.05 - 4.02 (m, 1H, *H*-2'), 3.00 (q, 12H, 2 x (NC*H*₂CH₃)₃), 1.47 (d, *J* = 5.5 Hz, 1.5H, NHCHC*H*₃), 1.36 (d, *J* = 5.5 Hz, 1.5H, NHCHC*H*₃),1.17 (t, *J* = 7.3 Hz, 18H, 2 x (NCH₂C*H*₃)₃). ³¹P NMR (202 MHz, CD₃OD): δ_{P} -7.53 (d, *J*_{P-P}= 18.90 Hz, 0.5P), -8.27 (d, *J*_{P-P} = 18.90 Hz, 0.5P), -11.66 (d, *J*_{P-P} = 20.60 Hz, 1P), -24.46 - (-24.68) (m, 1P).

### Example A: In vitro cytotoxicity analyses

A compound of the invention was assayed for cytotoxic activity in an array of different solid tumours and haematological malignancies using the following assay.

Solid tumour and haematological malignancy assay.

*In vitro* viability assay was performed to assess the effects of compounds on cell viability in selected cell lines over 72 h using the CellTiterGlo (CTG, Promega-G7573) assay. The tests were performed in duplicates with treatment of compounds at 9 points, 3.16 folds titration in 96 well plates over ~72 h. The compound starting concentrations were 198 µM. Cell viability assay using CellTiterGlo in 96-well plate were performed. Compound treatment 72 h, standard growth conditions, duplicates. Compounds were dissolved to 40mM with thawed 100%. Compounds were serially diluted at 3.16 fold in thawed DMSO, and warmed to 37°C before being dissolved in media (2µL+200µL). After compounds were dissolved in media, media containing compounds were warmed to 37°C in incubator and then compounds in media were added to cell plates (50µL+50µL) in duplicates. The compounds' final concentrations were from 198µM to 19.8nM. All compound solubilities were checked and recorded again, then the plates were transferred to CO₂ tissue culture incubator immediately and incubated for 3 days. DMSO final concentration is 0.5%.

The parent nucleoside drug was tested as a comparator, as was an exemplary monophosphate phosphoramidate A:

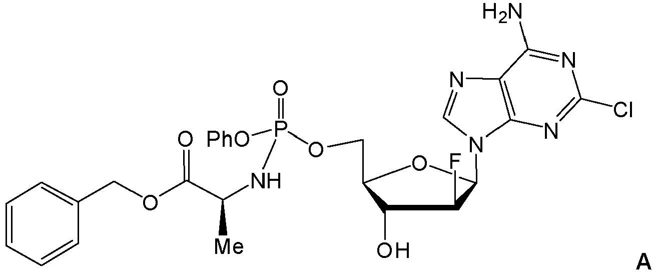

The following cell lines were tested and are referred to in the Table 1 below:

**Table 1**

| **Cell line** | **Malignancy** | **Cell line** | **Malignancy** |
|---|---|---|---|
| MOLT-4 | Acute T lymphoblastic leukaemia | MCF-7 | Breast adenocarcinoma |
| CCRF- CEM | Acute lymphoblastic leukaemia | HL-60 | Promyelocytic leukaemia |
| RL | Non-Hodgkin's lymphoma | HepG2 | Hepatocellular carcinoma |
| RPMI- 8226 | Human multiple myeloma | KG-1 | Acute myelogenous leukaemia |
| K562 | Chronic myelogenous leukaemia | HT29 | Colon adenocarcinoma |

The results of this screening are presented in Table 2 below:

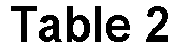

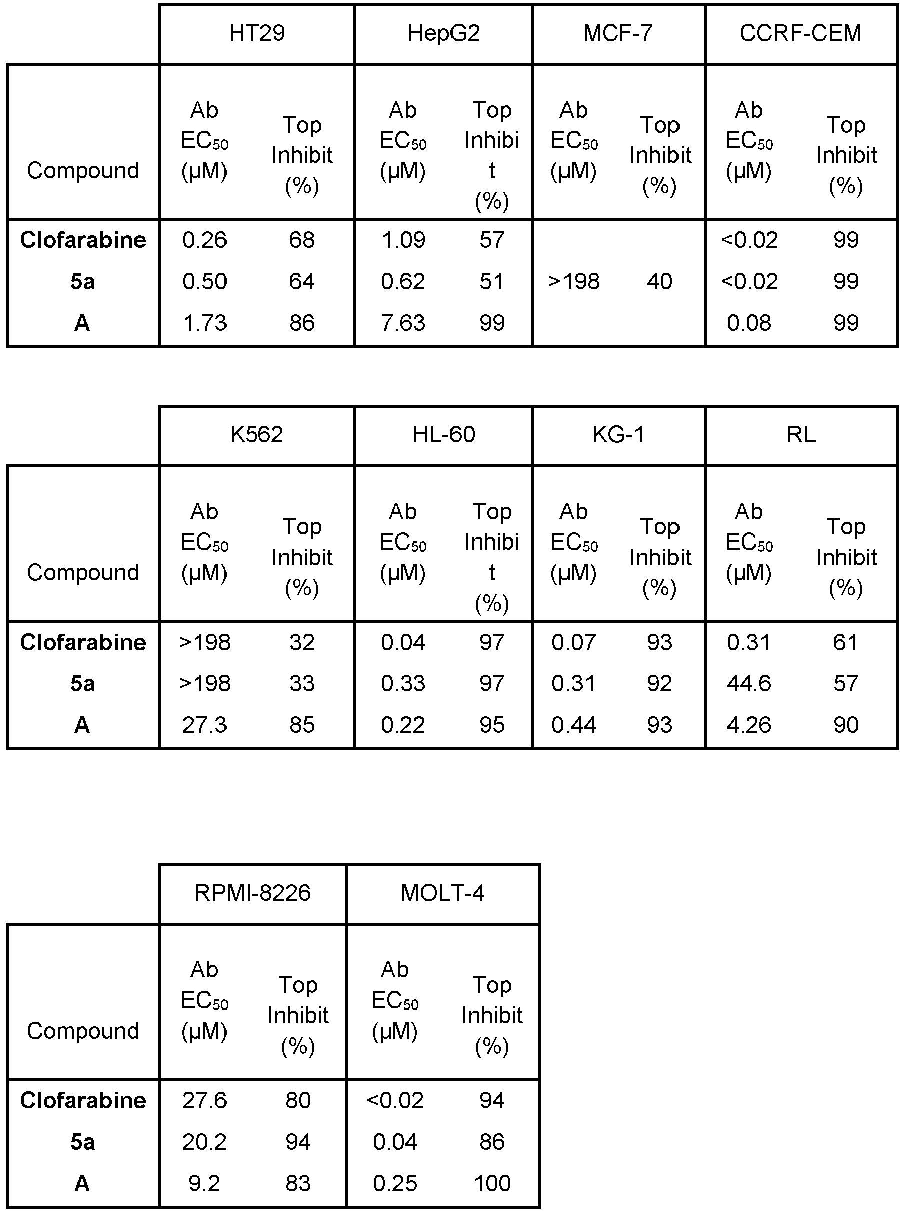

Against many cell lines, compound **5a** was more active than the corresponding monophosphate phosphoramidate.

### Example B: Efficacy evaluation of nucleoside analogue clofarabine and ProTides in cell lines model of acute leukaemia and lymphoma

The efficacy (evaluated by IC₅₀) of clofarabine and 3 different compounds representing different class of clofarabine ProTides (monophosphate, diphosphate and triphosphate molecules) was compared.

The efficacy of several triphosphate candidates compared to clofarabine and monophosphate was also evaluated.

The tested cancer cell lines were acute lymphoblastic leukaemia (ALL), acute myeloid leukaemia (AML) and lymphoma: CCRF-CEM (ALL), HL60 (AML), KG1 (AML), OCI-AML3 (AML) and RL (lymphoma), grown in suspension, in RPMI-1640 media, supplemented with 10% FBS serum and 1% pen strep, except for KG1 cell line for which FBS was 20%.

The parent nucleoside drug was tested as a comparator, as was an exemplary monophosphate phosphoramidate A and an exemplary diphosphate phosphoramidate **B**:

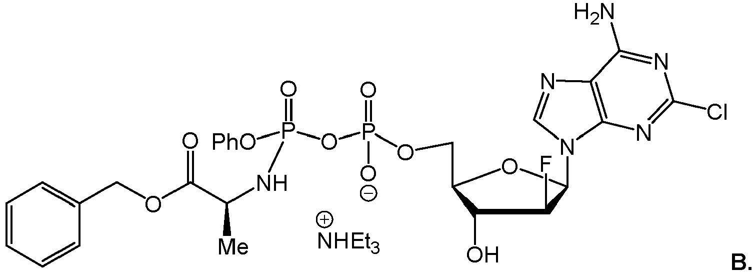

Compounds were dissolved in DMSO to a stock concentration of 10 mM, shown in Table 3 below.

**Table 3**

| Compound | MW (g/mol) | Weight (mg) | Concentration (mM) | Calculated volume (µL) | Solvent |
|---|---|---|---|---|---|
| **Clofarabine** | 303.7 | 2.52 | 10 | 829.77 | DMSO |
| **A** | 621 | 0.97 | 10 | 156.20 | DMSO |
| **B** | 802.12 | 16.5 | 10 | 2057.04 | DMSO |
| **5a** | 983.29 | 5.1 | 10 | 518.67 | DMSO |
| **5b** | 1033.35 | 0.82 | 10 | 79.35 | DMSO |
| **5c** | 935.3 | 1.81 | 10 | 187.12 | DMSO |
| **5d** | 967.31 | 1.13 | 10 | 116.82 | DMSO |
| **5e** | 1003.4 | 0.74 | 10 | 73.75 | DMSO |
| **5f** | 1039.4 | 0.71 | 10 | 68.31 | DMSO |

For all experiments, cells were plated on a 96 well plate, at a density of 10,000 cells/well in 50 µL media. Dilutions from stock concentration to working solutions were prepared in media; at twice the required concentration and 50 µL was added per well (this achieving the final concentrations described in results below). Control untreated received 50 µL of plain media. After 66h 10 µL of resazurin was added per well (dilution 1:10). Cells were then incubated with for up to 6h. After 72h treatment, cytotoxicity assay (AlamarBlueTM - resazurin dye) was performed to determine cell survival. Plates were scanned with a BioHit800 plate reader and data were analysed with Excel and GraphPad Prism.

### Comparison of clofarabine, A (monophosphate), B (diphosphate) and 5a (triphosphate) IC₅₀ in 5 different cell lines.

Cells were treated with serial dilution of each compounds (ranging from 6 nM to 6.3 µM) and the results represent the concentration that killed 50% of cells of each cell line, also called half maximal inhibitory concentration (IC₅₀). A low IC₅₀ indicates greater potency under these laboratory conditions. The results are shown in Table 4 below.

**Table 4**

| | IC₅₀ (nM) | | | | |
|---|---|---|---|---|---|
| Compound | CCRF-CEM | HL60 | KG1 | OCI-AML3 | RL |
| **Clofarabine** | 40.5 | 41.5 | 244 | 74 | 114 |
| **A** | 275 | 91 | 1761 | 253 | 399 |
| **B** | 82.5 | 71.9 | 267 | 121 | 153 |
| **5a** | 79 | 56.2 | 349 | 114 | 117 |

The diphosphate and triphosphate showed efficacy. IC₅₀ of **B** and **5a** were in the same comparable range as the parent nucleoside analogue (1.1 to 2-fold higher). **A** had the highest IC₅₀ in all of the cell lines tested (2.2 to 7.2-fold higher than clofarabine). For each of the cell lines, IC₅₀ of clofarabine was lower than the other compounds.

### Screen of multiple triphosphates against clofarabine in two cell lines.

**Clofarabine, A, 5a, 5b, 5c, 5d, 5e** and **5f** were tested against CCRF-CEM and OCI-AML3 cells. Data is represented as the percentage of cell survival for each of the different concentrations to which cells were exposed, rather than calculating a formal IC₅₀ from only two or three datapoints.

The results are shown in Table 5 below.

**Table 5**

| | CCRF-CEM | | |
|---|---|---|---|
| Compound | Survival @25 nM | Survival @ 50 nM | Survival @ 125 nM |
| **Clofarabine** | 36 | 17 | 13 |
| **A** | 96 | 100 | 100 |
| **5a** | 81 | 100 | 13 |
| **5b** | 36 | 18 | 13 |
| **5c** | 93 | 94 | 14 |
| **5d** | 92 | 16 | 14 |
| **5e** | 99 | 75 | 90 |
| **5f** | 85 | 16 | 14 |

| | OCI-AML3 | |
|---|---|---|
| Compound | Survival @ 50 nM | Survival @ 125 nM |
| **Clofarabine** | 47 | 19 |
| **A** | 99 | 96 |
| **5a** | 86 | 51 |
| **5b** | 64 | 24 |
| **5c** | 99 | 61 |
| **5d** | 61 | 29 |
| **5e** | 100 | 95 |
| **5f** | 57 | 32 |

Data in both cell lines showed superior efficacy of some triphosphate compounds (particularly **5b**, **5d** and **5f**) compared to **A**, and with efficacy relatively similar to clofarabine.

These data indicate that (1) several of the triphosphate compounds (which represent different families of molecules) are efficient in killing cancer cell lines comparable to the nucleoside analogues to which they are related and (2) in all cases better than the monophosphate ProTide **A**.

## Claims

1. A compound of formula (I), or a pharmaceutically acceptable salt thereof: 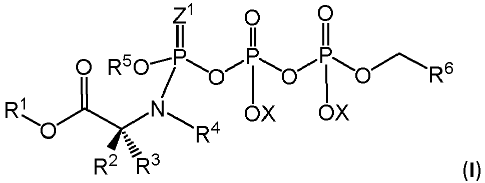 wherein
R¹ is independently at each occurrence selected from: C₁-C₂₄-alkyl, C₃-C₂₄-alkenyl, C₃-C₂₄-alkynyl, C₀-C₄-alkylene-C₃-C₈-cycloalkyl and C₀-C₄-alkylene-aryl;
R² and R³ are each independently at each occurrence selected from H, C₁-C₆-alkyl and C₁-C₃-alkylene-R⁷; or R² and R³ together with the atom to which they are attached form a 3- to 6-membered cycloalkyl or heterocycloalkyl group;
R⁴ is independently at each occurrence H or C₁-C₄-alkyl;
or R⁴, a group selected from R² and R³ and the atoms to which they are attached may form a 3- to 6-membered heterocycloalkyl group;
R⁵ is independently at each occurrence selected from aryl, 5-, 6-, 9- or 10-membered heteroaryl, C₃-C₈-cycloalkyl, 3- to 7-membered heterocycloalkyl, C₁-C₃-alkylene-R^{5a} and C₁-C₈-alkyl, said aryl being optionally fused to C₆-C₈-cycloalkyl;
R^{5a} is independently at each occurrence selected from aryl, 5-, 6-, 9- or 10-membered heteroaryl, C₃-C₈-cycloalkyl, 3- to 7-membered heterocycloalkyl, said aryl being optionally fused to C₆-C₈-cycloalkyl;
R⁶ is independently selected from: 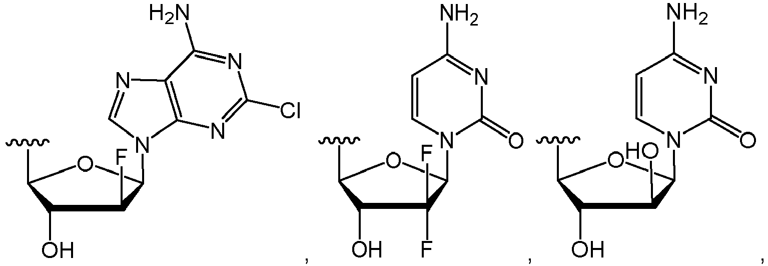 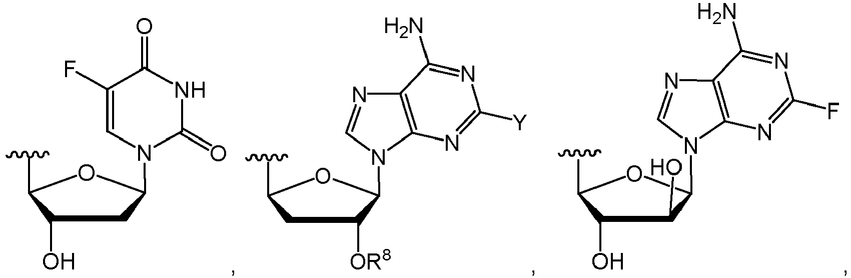 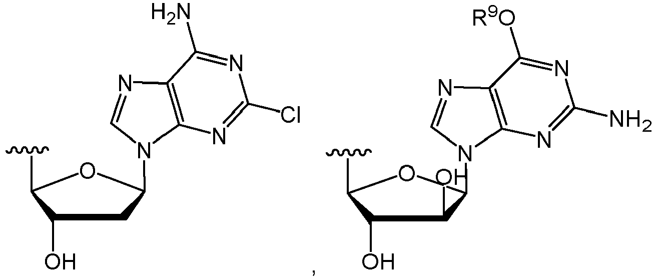 and 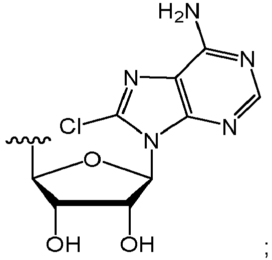
R⁷ is independently at each occurrence selected from aryl, imidazole, indole, SR^{a}, OR^{a}, CO₂R^{a}, CO₂NR^{a}R^{a}, NR^{a}R^{b} and NH(=NH)NH₂;
R⁸ is independently selected from H and 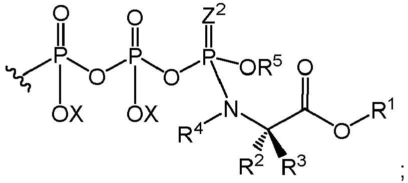
Z¹ and Z² are each independently selected from O and S;
R⁹ is independently selected from H and Me;
Y is independently selected from H, F, Cl and OMe;
X is at each occurrence selected from H and a pharmaceutically acceptable cation; provided that in at least one occurrence X is a pharmaceutically acceptable cation;
wherein any aryl group is either phenyl or naphthyl;
wherein where any of R¹, R², R³, R⁴, R⁵ or R⁷ is an alkyl, cycloalkyl, heterocycloalkyl, aryl or heteroaryl, that alkyl, cycloalkyl, heterocycloalkyl, aryl or heteroaryl group is optionally substituted with from 1 to 4 substituents selected from: halo, nitro, cyano, NR^{a}R^{a}, NR^{a}S(O)₂R^{a}, NR^{a}C(O)R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, CR^{a}R^{a}NR^{a}R^{a}, C₁-C₄-alkyl, C₂-C₄-alkenyl, C₂-C₄-alkynyl and C₁-C₄-haloalkyl;
wherein R^{a} is independently at each occurrence selected from: H and C₁-C₄-alkyl; and R^{b} is independently at each occurrence selected from: H, and C₁-C₄-alkyl and C(O)-C₁-C₄-alkyl.

2. A compound of claim 1, wherein R⁴ is H.

3. A compound of claim 1 or claim 2, wherein R⁶ is

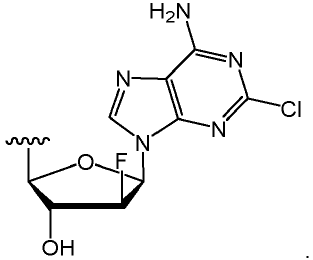

4. A compound of claim 1 or claim 2, wherein R⁶ is 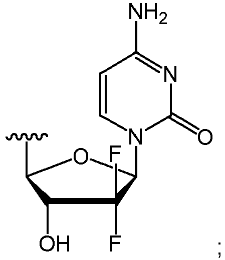 or
wherein R⁶ is 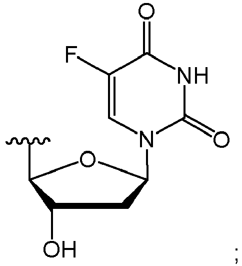 or
wherein R⁶ is 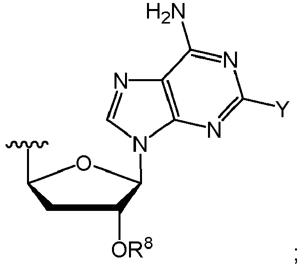 or
wherein R⁶ is 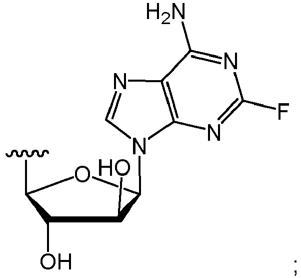 or
wherein R⁶ is 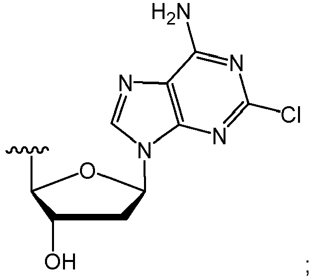 or
wherein R⁶ is 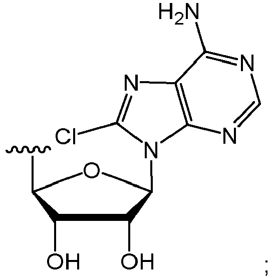 or
wherein R⁶ is

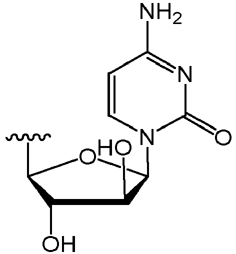

5. A compound of any one of claims 1 to 4, wherein R¹ is selected from C₅-C₇-cycloalkyl, C₁-C₈-alkyl and benzyl.

6. A compound of claim 5, wherein R¹ is benzyl.

7. A compound of claim 5, wherein R¹ is C₁-C₈-alkyl; optionally wherein R¹ is ethyl.

8. A compound of any one of claims 1 to 7, wherein R³ is H.

9. A compound of any one of claims 1 to 8, wherein R² is C₁-C₄-alkyl.

10. A compound of any one of claims 1 to 8, wherein R² is H.

11. A compound of any one of claims 1 to 10, wherein R⁵ is phenyl.

12. A compound of any one of claims 1 to 10, wherein R⁵ is naphthyl.

13. A compound of claim 1, wherein the compound of formula (I) is selected from:

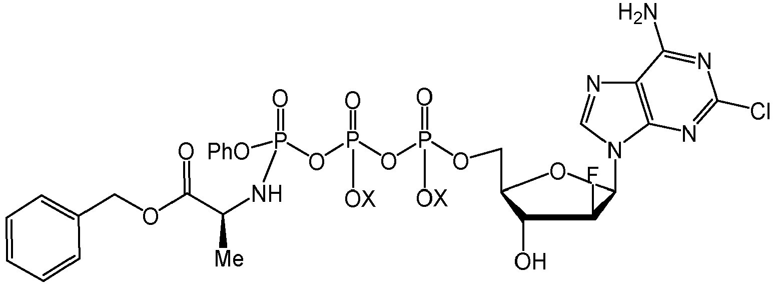

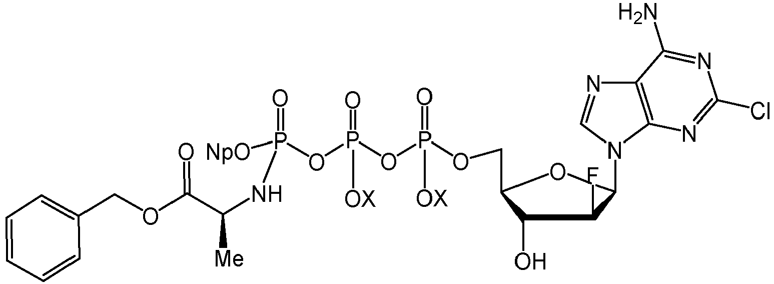

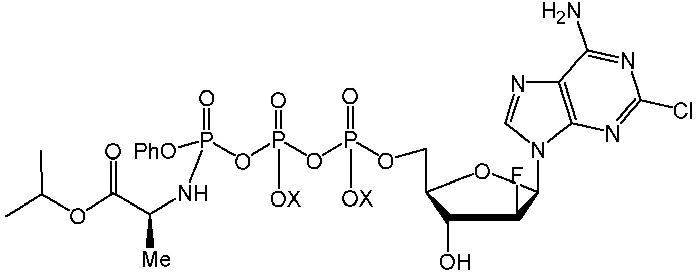

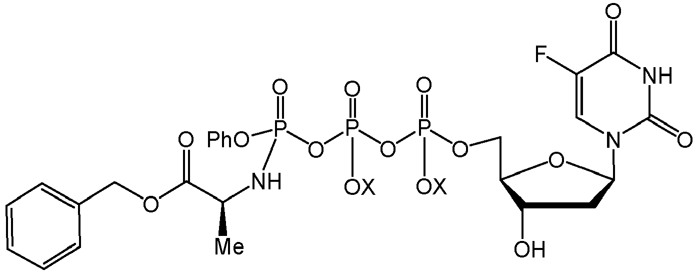

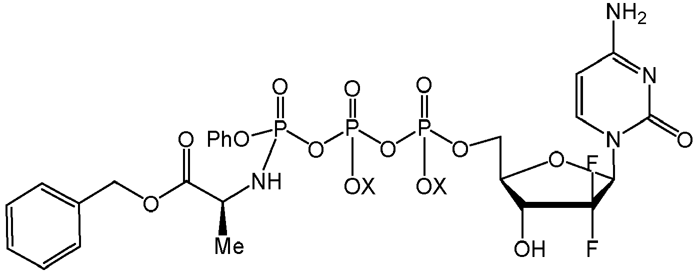

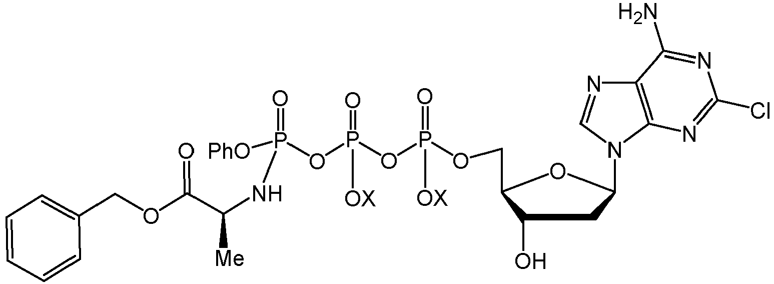

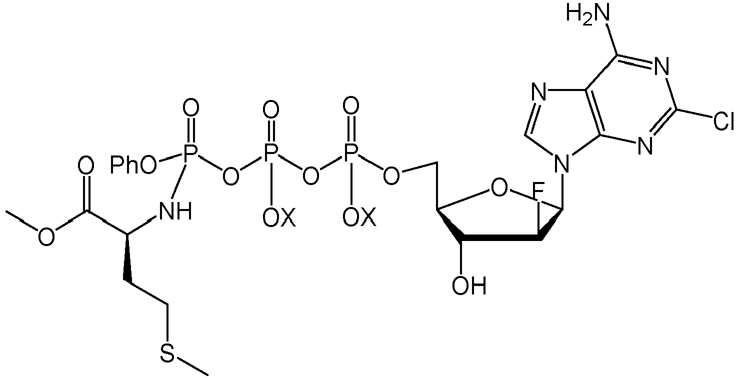

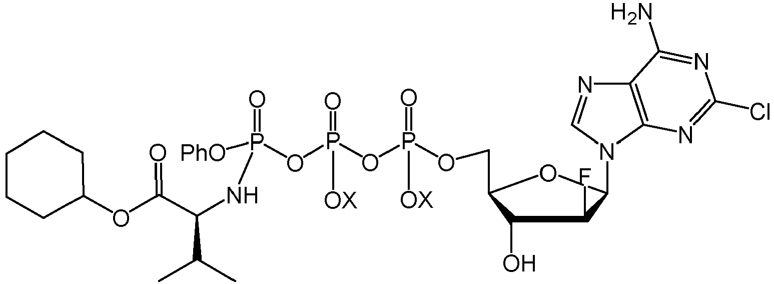

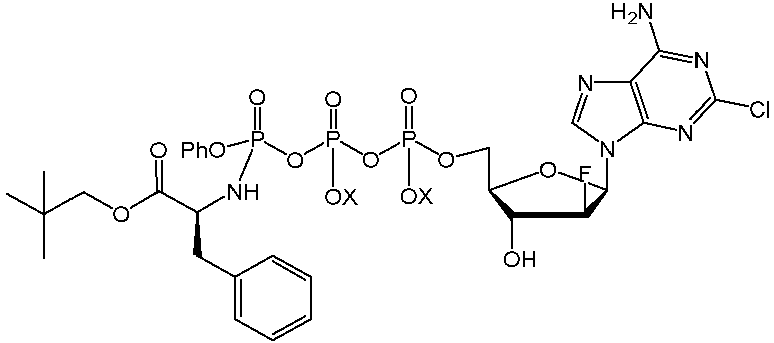

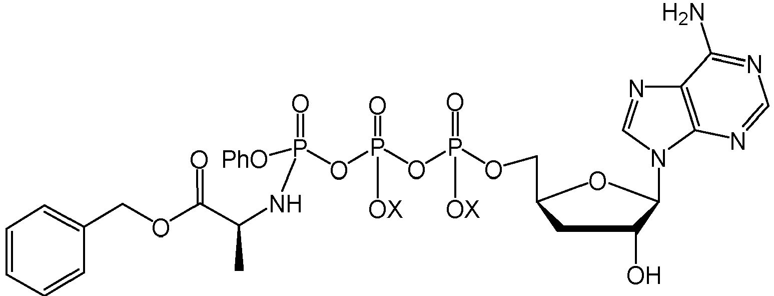

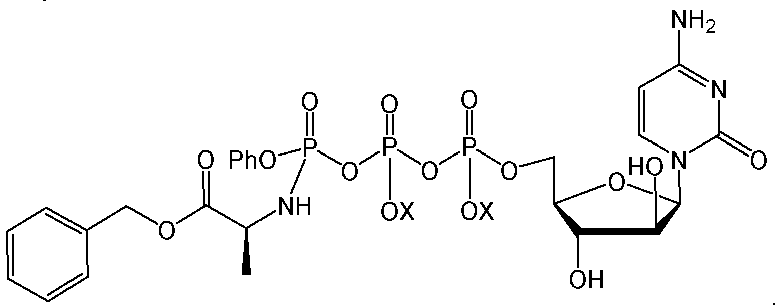

14. A compound of any one of claims 1 to 13, wherein X is at one occurrence H and at one occurrence a metal cation or an ammonium cation; or
wherein X is at each occurrence the same and is a metal cation or an ammonium cation.

15. A compound of any one of claims 1 to 14 for medical use.

16. A compound of any one of claims 1 to 14 for use in treating cancer; optionally wherein the cancer is leukaemia or lymphoma; further optionally
wherein the cancer is a leukaemia selected from the group consisting of acute myeloid leukaemia (AML), acute lymphoid leukaemia (ALL), chronic myeloid leukaemia (CML), chronic lymphoid leukaemia (CLL) and biphenotypic acute leukaemia (BAL); or
wherein the cancer is a lymphoma selected from the group consisting of Hodgkin's lymphoma and non-Hodgkin's lymphoma.

17. A pharmaceutical composition comprising compounds of any one of claims 1 to 14 and at least one pharmaceutically acceptable excipient.

## Patentansprüche

1. Verbindung der Formel (I) oder pharmazeutisch unbedenkliches Salz davon: 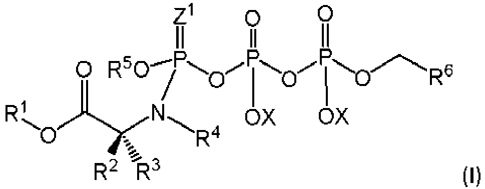 wobei
R¹ unabhängig bei jedem Auftreten ausgewählt ist aus: C₁-C₂₄-Alkyl, C₃-C₂₄-Alkenyl, C₃-C₂₄-Alkynyl, C₀-C₄-Alkylen-C₃-C₈-cycloalkyl und C₀-C₄-Alkylenaryl;
R² und R³ jeweils unabhängig bei jedem Auftreten ausgewählt sind aus H, C₁-C₆-Alkyl und C₁-C₃-Alkylen-R⁷; oder R² und R³ zusammen mit dem Atom, an das sie gebunden sind, eine 3- bis 6-gliedrige Cycloalkyl- oder Heterocycloalkylgruppe bilden;
R⁴ unabhängig bei jedem Auftreten H oder C₁-C₄-Alkyl ist;
oder R⁴, eine Gruppe ausgewählt aus R² und R³ und die Atome, an die sie gebunden sind, eine 3- bis 6-gliedrige Heterocycloalkylgruppe bilden können;
R⁵ unabhängig bei jedem Auftreten ausgewählt ist aus Aryl, 5-, 6-, 9- oder 10-gliedrigem Heteroaryl, C₃-C₈-Cycloalkyl, 3- bis 7-gliedrigem Heterocycloalkyl, C₁-C₃-Alkylen-R^{5a} und C₁-C₈-Alkyl, wobei das Aryl optional an C₆-C₈-Cycloalkyl kondensiert ist;
R^{5a} unabhängig bei jedem Auftreten ausgewählt ist aus Aryl, 5-, 6-, 9- oder 10-gliedrigem Heteroaryl, C₃-C₈-Cycloalkyl, 3- bis 7-gliedrigem Heterocycloalkyl, wobei das Aryl optional an C₆-C₈-Cycloalkyl kondensiert ist;
R⁶ unabhängig ausgewählt ist aus: 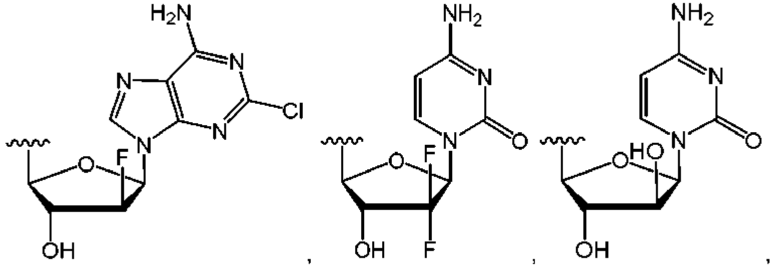 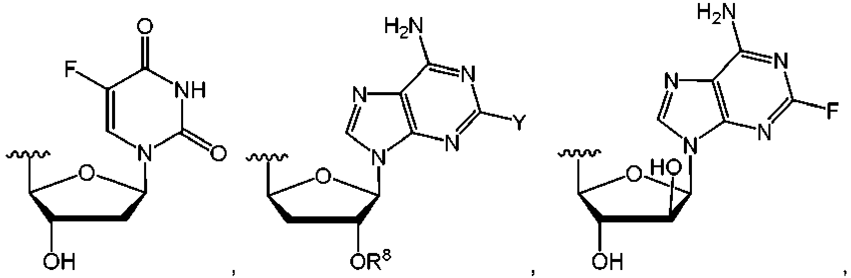 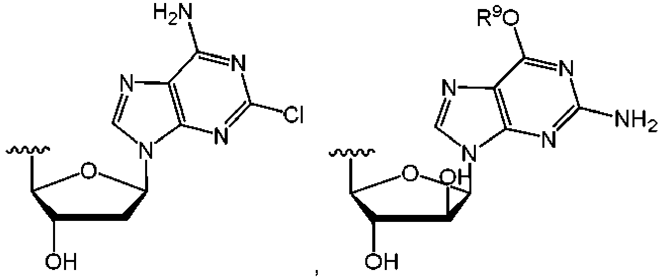 und 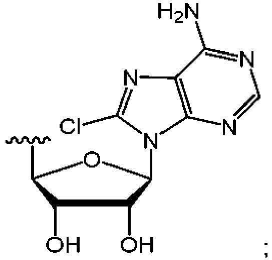
R⁷ unabhängig bei jedem Auftreten ausgewählt ist aus Aryl, Imidazol, Indol, SR^{a}, OR^{a}, CO₂R^{a}, CO₂NR^{a}R^{a}, NR^{a}R^{b} und NH(=NH)NH₂;
R⁸ unabhängig ausgewählt ist aus H und 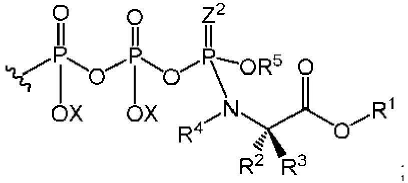
Z¹ und Z² jeweils unabhängig ausgewählt sind aus O und S;
R⁹ unabhängig ausgewählt ist aus H und Me;
Y unabhängig ausgewählt ist aus H, F, Cl und OMe;
X bei jedem Auftreten ausgewählt ist aus H und einem pharmazeutisch unbedenklichen Kation; vorausgesetzt, dass bei mindestens einem Auftreten X ein pharmazeutisch unbedenkliches Kation ist;
wobei jede Arylgruppe entweder Phenyl oder Naphthyl ist;
wobei, wenn eines von R¹, R², R³, R⁴, R⁵ oder R⁷ ein Alkyl, Cycloalkyl, Heterocycloalkyl, Aryl oder Heteroaryl ist, diese Alkyl-, Cycloalkyl-, Heterocycloalkyl-, Aryl- oder Heteroarylgruppe optional mit 1 bis 4 Substituenten substituiert ist, die ausgewählt sind aus: Halo, Nitro, Cyano, NR^{a}R^{a}, NR^{a}S(O)₂R^{a}, NR^{a}C(O)R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, CR^{a}R^{a}NR^{a}R^{a}, C₁-C₄-Alkyl, C₂-C₄-Alkenyl, C₂-C₄-Alkynyl und C₁-C₄-Haloalkyl;
wobei R^{a} unabhängig bei jedem Auftreten ausgewählt ist aus: H und C₁-C₄-Alkyl; und R^{b} unabhängig bei jedem Auftreten ausgewählt ist aus: H und C₁-C₄-Alkyl und C(O)-C₁-C₄-Alkyl.

2. Verbindung nach Anspruch 1, wobei R⁴ H ist.

3. Verbindung nach Anspruch 1 oder Anspruch 2, wobei R⁶ Folgendes ist

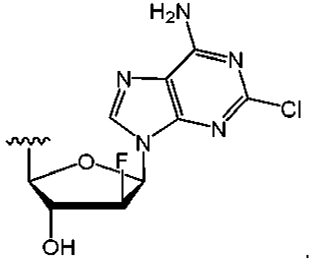

4. Verbindung nach Anspruch 1 oder Anspruch 2, wobei R⁶ Folgendes ist 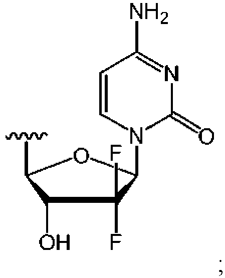 oder
wobei R⁶ Folgendes ist 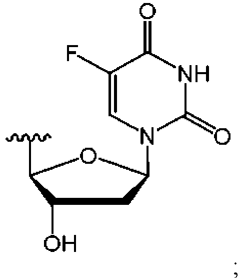 oder
wobei R⁶ Folgendes ist 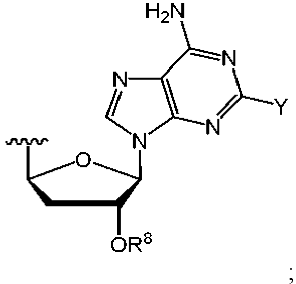 oder
wobei R⁶ Folgendes ist 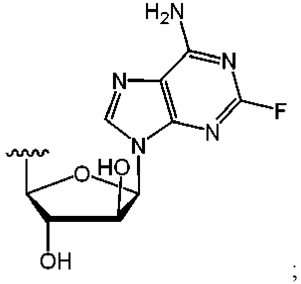 oder
wobei R⁶ Folgendes ist 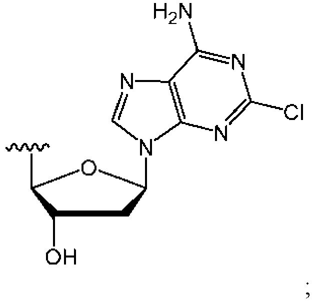 oder
wobei R⁶ Folgendes ist 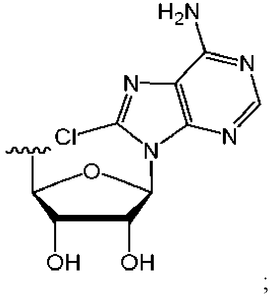 oder
wobei R⁶ Folgendes ist

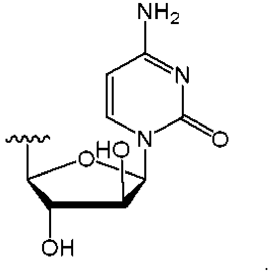

5. Verbindung nach einem der Ansprüche 1 bis 4, wobei R¹ ausgewählt ist aus C₅-C₇-Cycloalkyl, C₁-C₈-Alkyl und Benzyl.

6. Verbindung nach Anspruch 5, wobei R¹ Benzyl ist.

7. Verbindung nach Anspruch 5, wobei R¹ C₁-C₈-Alkyl ist; optional wobei R¹ Ethyl ist.

8. Verbindung nach einem der Ansprüche 1 bis 7, wobei R³ H ist.

9. Verbindung nach einem der Ansprüche 1 bis 8, wobei R² C₁-C₄-Alkyl ist.

10. Verbindung nach einem der Ansprüche 1 bis 8, wobei R² H ist.

11. Verbindung nach einem der Ansprüche 1 bis 10, wobei R⁵ Phenyl ist.

12. Verbindung nach einem der Ansprüche 1 bis 10, wobei R⁵ Naphthyl ist.

13. Verbindung nach Anspruch 1, wobei die Verbindung der Formel (I) ausgewählt ist aus:

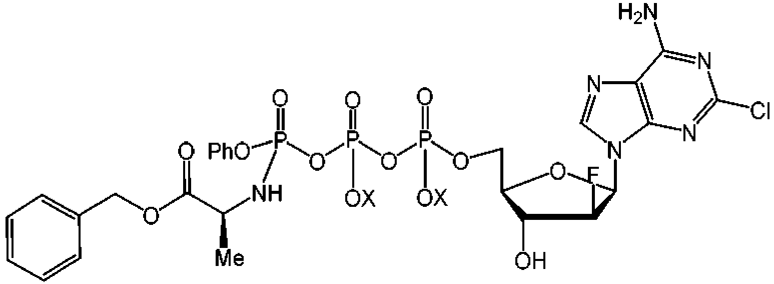

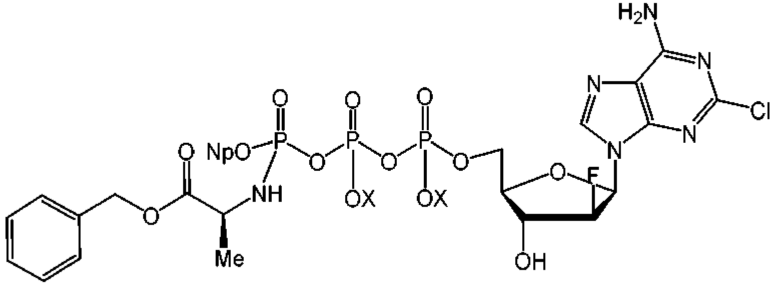

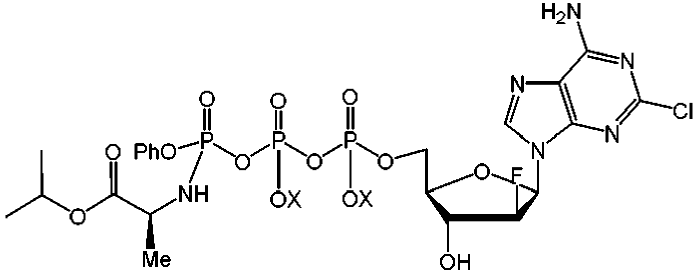

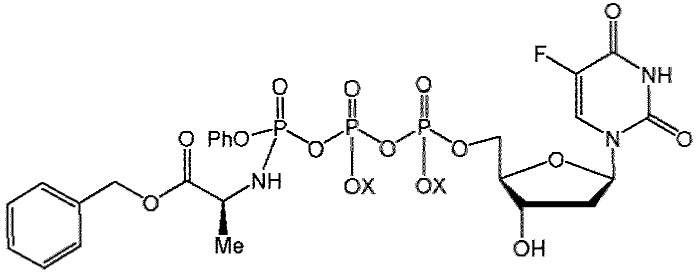

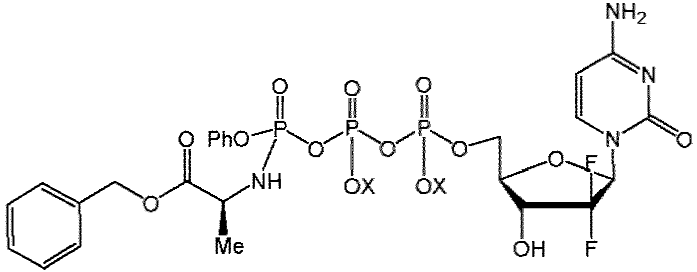

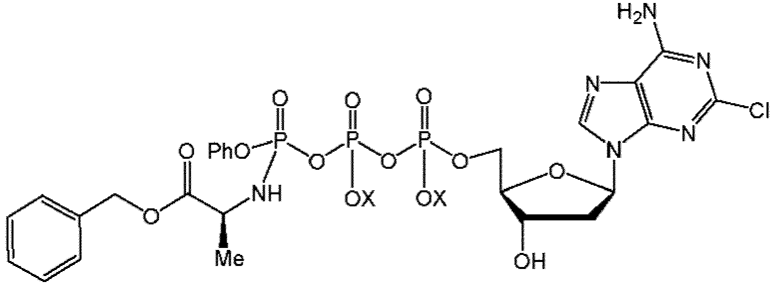

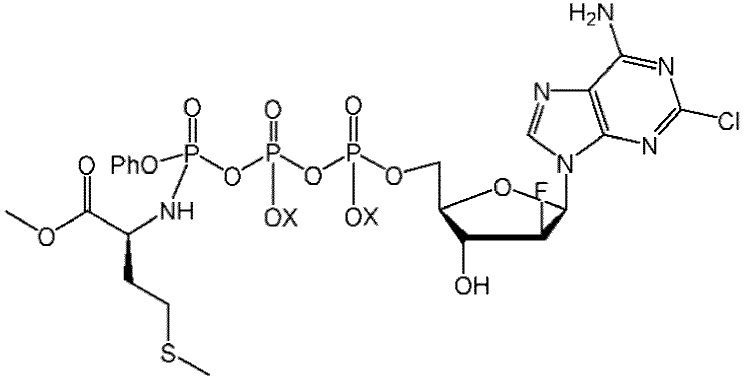

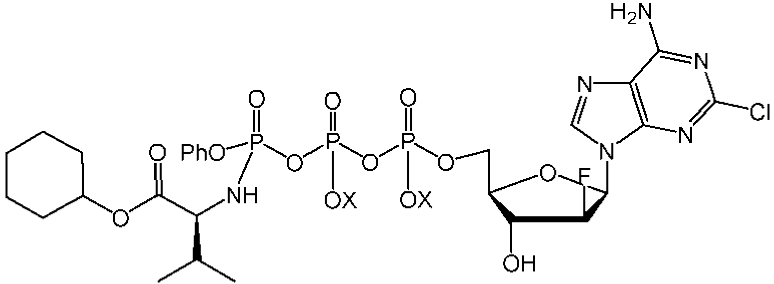

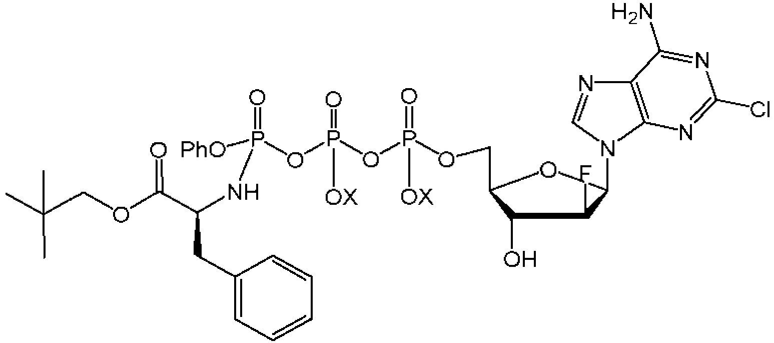

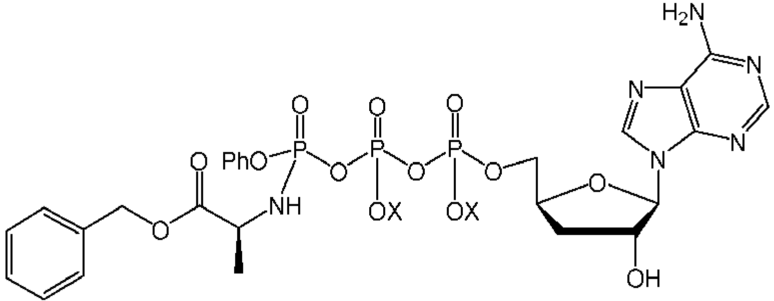

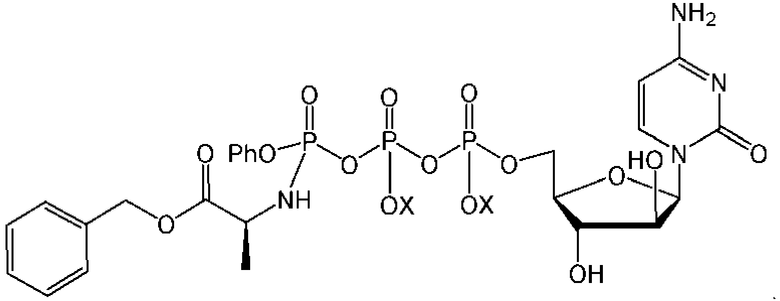

14. Verbindung nach einem der Ansprüche 1 bis 13, wobei X bei einem Auftreten H und bei einem Auftreten ein Metallkation oder ein Ammoniumkation ist; oder
wobei X bei jedem Auftreten gleich ist und ein Metallkation oder ein Ammoniumkation ist.

15. Verbindung nach einem der Ansprüche 1 bis 14 zur medizinischen Verwendung.

16. Verbindung nach einem der Ansprüche 1 bis 14 zur Verwendung bei der Behandlung von Krebs; wobei der Krebs optional Leukämie oder ein Lymphom ist; ferner optional
wobei der Krebs eine Leukämie ist, die aus der Gruppe ausgewählt ist, die aus akuter myeloischer Leukämie (AML), akuter lymphatischer Leukämie (ALL), chronischer myeloischer Leukämie (CML), chronischer lymphatischer Leukämie (CLL) und biphänotypischer akuter Leukämie (BAL) besteht; oder
wobei der Krebs ein Lymphom ist, das aus der Gruppe ausgewählt ist, die aus Hodgkin-Lymphom und Non-Hodgkin-Lymphom besteht.

17. Pharmazeutische Zusammensetzung, umfassend Verbindungen nach einem der Ansprüche 1 bis 14 und mindestens einen pharmazeutisch unbedenklichen Hilfsstoff.

## Revendications

1. Composé de formule (I), ou sel de celui-ci de qualité pharmaceutique : 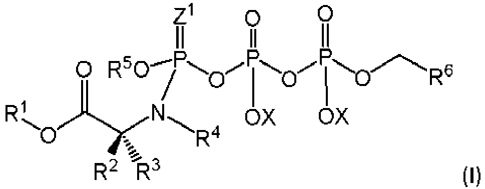 où
R¹ est, indépendamment à chaque occurrence, sélectionné entre : C₁-C₂₄-alkyle, C₃-C₂₄-alkényle, C₃-C₂₄-alkynyle, C₀-C₄-alkylène-C₃-C₈-cycloalkyle et C₀-C₄-alkylène-aryle ;
R² et R³ sont, chacun indépendamment à chaque occurrence, sélectionnés entre H, C₁-C₆-alkyle et C₁-C₃-alkylène-R⁷ ; ou R² et R³, conjointement avec l'atome auquel ils sont fixés, forment un groupe cycloalkyle ou hétérocycloalkyle à 3 à 6 chaînons ;
R⁴ est, indépendamment à chaque occurrence, H ou C₁-C₄-alkyle ;
ou R⁴, un groupe sélectionné entre R² et R³ et les atomes auxquels ils sont fixés peuvent former un groupe hétérocycloalkyle à 3 à 6 chaînons ;
R⁵ est, indépendamment à chaque occurrence, sélectionné entre aryle, hétéroaryle à 5, 6, 9 ou 10 chaînons, C₃-C₈-Cycloalkyle, hétérocycloalkyle à 3 à 7 chaînons, C₁-C₃-alkylène-R^{5a} et C₁-C₈-alkyle, ledit aryle étant optionnellement fusionné à C₆-C₈-cycloalkyle ;
R^{5a} est, indépendamment à chaque occurrence, sélectionné entre aryle, hétéroaryle à 5, 6, 9 ou 10 chaînons, C₃-C₈-cycloalkyle, hétérocycloalkyle à 3 à 7 chaînons, ledit aryle étant optionnellement fusionné à C₆-C₈-cycloalkyle ;
R⁶ est indépendamment sélectionné entre : 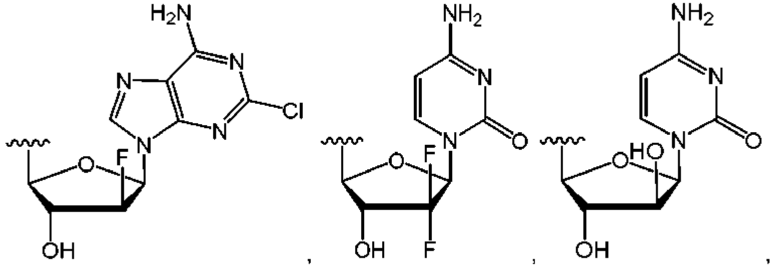 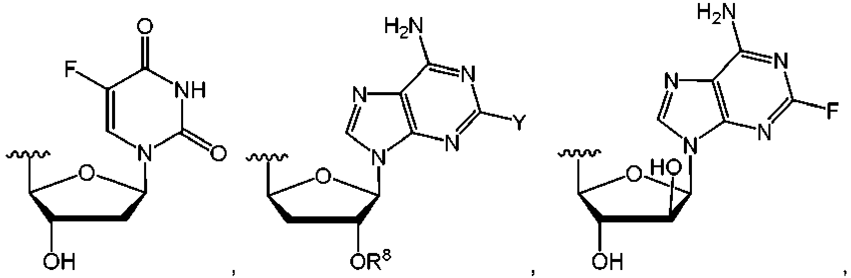 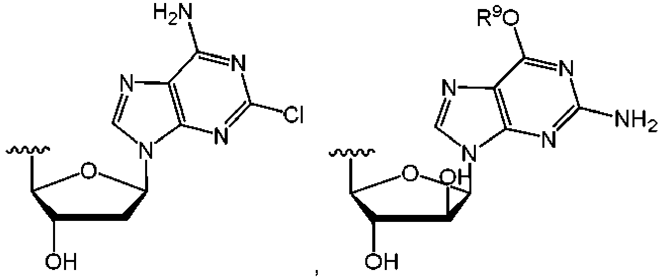 et 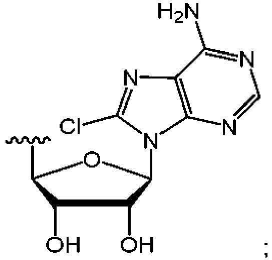
R⁷ est, indépendamment à chaque occurrence, sélectionné entre aryle, imidazole, indole, SR^{a}, OR^{a}, CO₂R^{a}, CO₂NR^{a}R^{a}, NR^{a}R^{b} et NH(=NH)NH₂ ;
R⁸ est indépendamment sélectionné entre H et 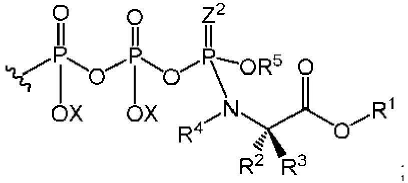
Z¹ et Z² sont chacun indépendamment sélectionnés entre O et S ;
R⁹ est indépendamment sélectionné entre H et Me ;
Y est indépendamment sélectionné entre H, F, Cl et OMe ;
X est, à chaque occurrence, sélectionné entre H et un cation de qualité pharmaceutique ; étant entendu que, dans au moins une occurrence, X est un cation de qualité pharmaceutique ;
où un groupe aryle quelconque est soit phényle soit naphthyle ;
où l'un quelconque de R¹, R², R³, R⁴, R⁵ ou R⁷ est un alkyle, cycloalkyle, hétérocycloalkyle, aryle ou hétéroaryle, ce groupe alkyle, cycloalkyle, hétérocycloalkyle, aryle ou hétéroaryle est optionnellement substitué par 1 à 4 substituants sélectionnés entre : halo, nitro, cyano, NR^{a}R^{a}, NR^{a}S(O)₂R^{a}, NR^{a}C(O)R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, CR^{a}R^{a}NR^{a}R^{a}, C₁-C₄-alkyle, C₂-C₄-alkényle, C₂-C₄-alkynyle et C₁-C₄-haloalkyle ;
où R^{a} est, indépendamment à chaque occurrence, sélectionné entre : H et C₁-C₄-alkyle ; et R^{b} est, indépendamment à chaque occurrence, sélectionné entre : H, C₁-C₄-alkyle et C(O)-C₁-C₄-alkyle.

2. Composé selon la revendication 1, où R⁴ est H.

3. Composé selon la revendication 1 ou la revendication 2, où R⁶ est

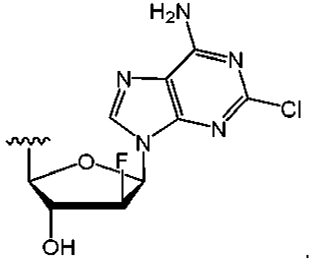

4. Composé selon la revendication 1 ou la revendication 2, où R⁶ est 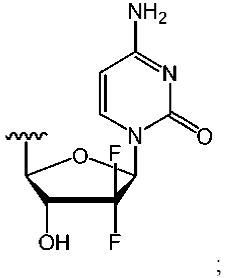 ou
où R⁶ est 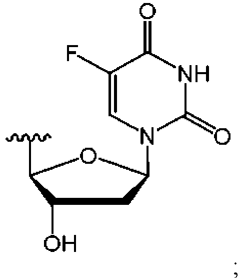 ou
où R⁶ est 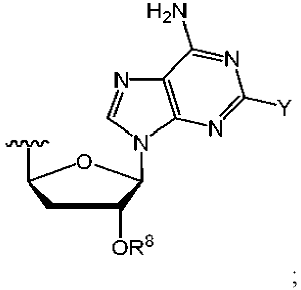 ou
où R⁶ est 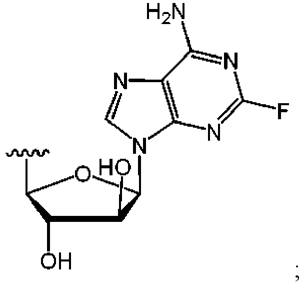 ou
où R⁶ est 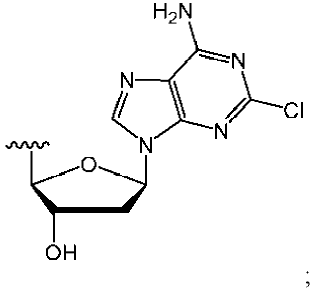 ou
où R⁶ est 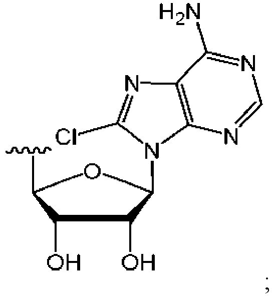 ou
où R⁶ est

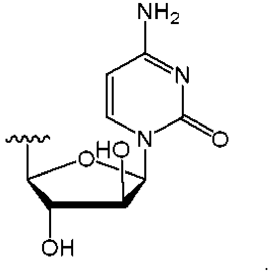

5. Composé selon l'une quelconque des revendications 1 à 4, où R¹ est sélectionné entre C₅-C₇-cycloalkyle, C₁-C₈-alkyle et benzyle.

6. Composé selon la revendication 5, où R¹ est benzyle.

7. Composé selon la revendication 5, où R¹ est C₁-C₈-alkyle ; optionnellement où R¹ est éthyle.

8. Composé selon l'une quelconque des revendications 1 à 7, où R³ est H.

9. Composé selon l'une quelconque des revendications 1 à 8, où R² est C₁-C₄-alkyle.

10. Composé selon l'une quelconque des revendications 1 à 8, où R² est H.

11. Composé selon l'une quelconque des revendications 1 à 10, où R⁵ est phényle.

12. Composé selon l'une quelconque des revendications 1 à 10, où R⁵ est naphthyle.

13. Composé selon la revendication 1, où le composé de formule (I) est sélectionné entre :

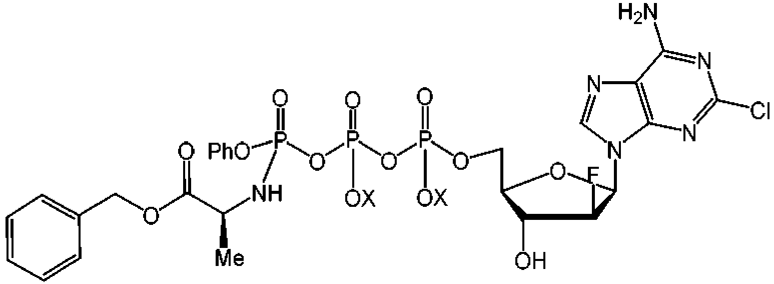

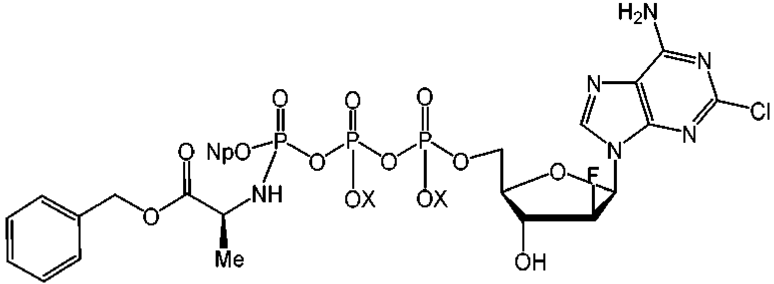

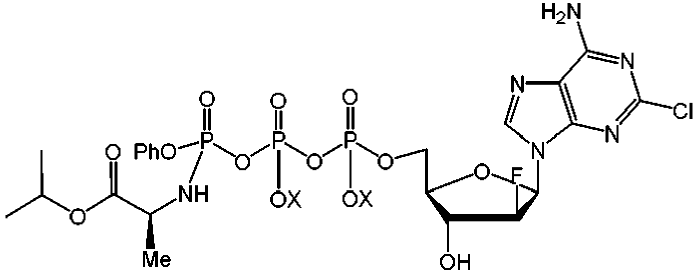

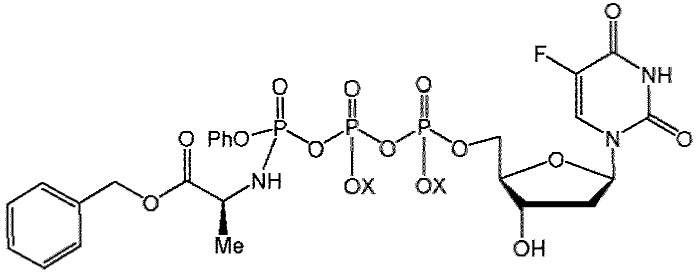

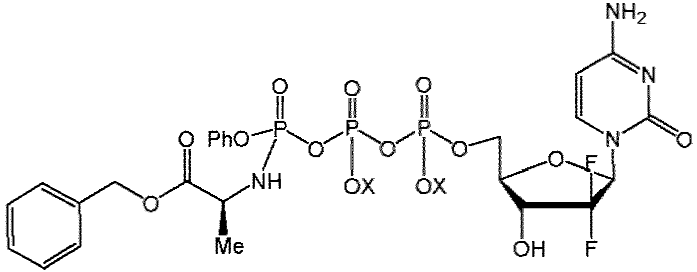

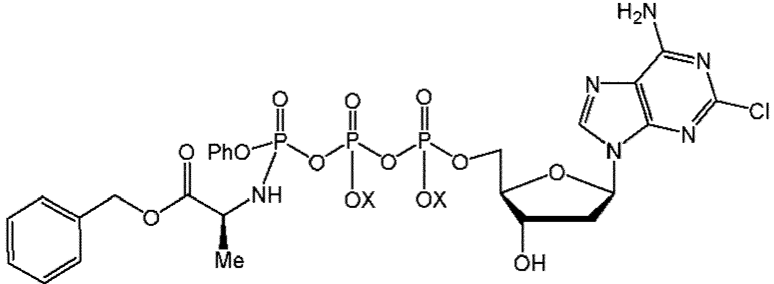

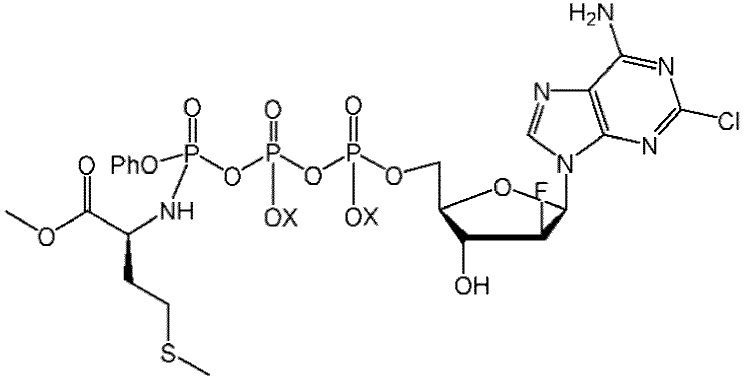

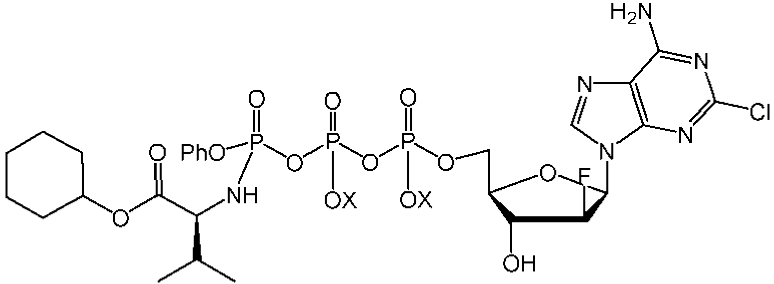

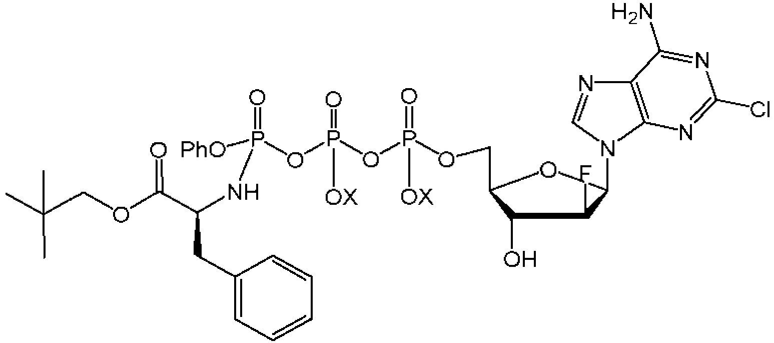

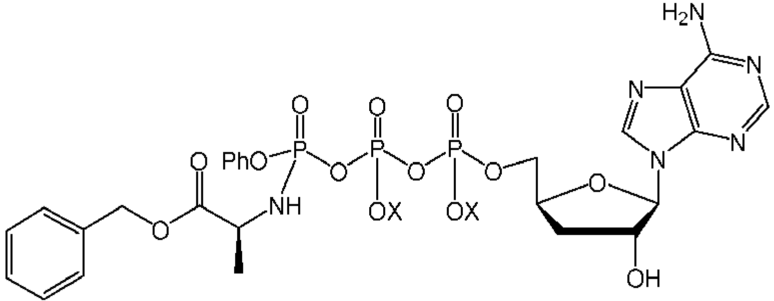

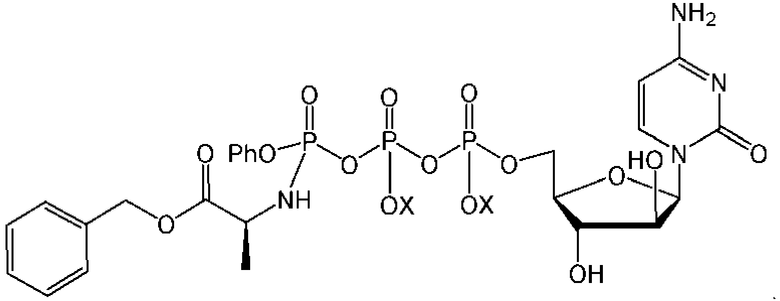

14. Composé selon l'une quelconque des revendications 1 à 13, où X est H à une occurrence, et un cation métallique ou un cation d'ammonium à une occurrence ; ou
où X est le même à chaque occurrence et est un cation métallique ou un cation d'ammonium.

15. Composé selon l'une quelconque des revendications 1 à 14 pour utilisation médicale.

16. Composé selon l'une quelconque des revendications 1 à 14 pour utilisation dans le traitement d'un cancer ; optionnellement où le cancer est une leucémie ou un lymphome ; en outre, optionnellement
où le cancer est une leucémie sélectionnée dans le groupe constitué de leucémie myéloïde aiguë (LMA), leucémie lymphoïde aiguë (LLA), leucémie myéloïde chronique (LMC), leucémie lymphoïde chronique (LLC) et leucémie biphénotypique aiguë (LBA) ; ou
où le cancer est un lymphome sélectionné dans le groupe constitué de lymphome hodgkinien et lymphome non hodgkinien.

17. Composition pharmaceutique comprenant des composés selon l'une quelconque des revendications 1 à 14 et au moins un excipient de qualité pharmaceutique.
